# EUROPÄISCHE PATENTSCHRIFT

(11) **EP 1 803 699 B1**
(45) Veröffentlichungstag und Bekanntmachung des Hinweises auf die Patenterteilung: **13.03.2013**
(21) Anmeldenummer: 06126458.6
(22) Anmeldetag: 19.12.2006
(51) Int. Cl.: C07C 7/148, C07C 11/08

(54) **Verfahren zur Feinreinigung von 1-Buten-haltigen-Strömen**
Method for fine cleaning of 1-buten flows
Procédé pour l'épuration poussée de flux contenant du butène-1

(30) Priorität: 28.12.2005 DE 102005062699
(43) Veröffentlichungstag der Anmeldung: 04.07.2007
(73) Patentinhaber: Evonik Oxeno GmbH, 45772 Marl (DE)
(72) Erfinder: Santiago Fernandez, Silvia, 46049, Oberhausen (DE); Rix, Armin, 45770, Marl (DE); Praefke, Jochen, 45739, Oer-Erkenschwick (DE); Röttger, Dirk, 45657, Recklinghausen (DE); Winterberg, Markus, 45711, Datteln (DE); Büschken, Wilfried, 45721, Haltern am See (DE)
(74) Vertreter: Hirsch, Hans-Ludwig

(56) Entgegenhaltungen:
- EP-A1- 1 199 296
- WO-A-2004/065338
- DE-A1- 2 521 964
- DE-A1- 2 853 769
- DATABASE WPI Week 200146 Derwent Publications Ltd., London, GB; AN 2001-431147 XP002435238 & RU 2 168 490 C1 (YARSINTEZ SCI PRODN INST STOCK CO) 10. Juni 2001 (2001-06-10)

## Beschreibung

Die Erfindung betrifft ein Verfahren zur Feinreinigung von 1-Buten-haltigen Strömen, die mehr als 2000 wppm (Massen-ppm) und weniger als 8 Massen-% Isobuten aufweisen.

1-Buten, Isobuten, 2-Butene und deren Folgeprodukte werden in großen Mengen aus technischen C₄-Schnitten, beispielsweise dem C₄-Schnitt aus Steamcrackern oder FCC-Einheiten, gewonnen. Diese Gemische bestehen im Wesentlichen aus Butadien, den Monoolefinen Isobuten, 1-Buten und den beiden 2-Butenen sowie den gesättigten Kohlenwasserstoffen Isobutan und n-Butan. Wegen der geringen Siedepunktunterschiede der Inhaltsstoffe und deren geringen Trennfaktoren ist eine destillative Aufarbeitung schwierig und nicht wirtschaftlich. Die Gewinnung von linearen Butenen und von anderen Produkten erfolgt daher meistens durch eine Kombination von chemischen Umsetzungen und physikalischen Trennoperationen.

Der erste Schritt, den dabei alle Aufarbeitungsvarianten gemeinsam haben, ist die Entfernung des größten Teils des Butadiens. Kann Butadien gut vermarktet werden oder besteht ein Eigenverbrauch, wird es üblicherweise durch Extraktion oder Extraktivdestillation abgetrennt. Im anderen Fall wird es bis zu einer Restkonzentration von circa 2000 Massen-ppm selektiv zu linearen Butenen hydriert. Zurück bleibt in beiden Fällen ein Kohlenwasserstoffgemisch (sogenanntes Raffinat I oder selektiv hydriertes Crack-C₄), das neben den gesättigten Kohlenwasserstoffen n-Butan und Isobutan die Olefine Isobuten, 1-Buten und 2-Butene (cis und trans) enthält.

Für die Gewinnung von 1-Buten als Zielprodukt wird üblicherweise wie folgt vorgegangen: Aus Raffinat I oder hydriertem Crack-C₄ wird durch chemische Umsetzung Isobuten weitestgehend entfernt. Nach der weitgehenden Entfernung des Isobutens bleibt ein Kohlenwasserstoffgemisch (Raffinat II), das die linearen Butene und die gesättigten Kohlenwasserstoffe Isobutan und n-Butan enthält und das destillativ weiter aufgetrennt werden kann. Um das 1-Buten in der Ethylenpolymerisation, in der Isobuten-Verunreinigungen unerwünscht sind, einsetzen zu können, sollte ein spezifikationsgerechtes 1-Buten einen Gehalt an Isobuten von kleiner 2000 ppm bezogen auf das 1-Buten aufweisen.

Eine weit verbreitete Möglichkeit zur chemischen Umsetzung von Isobuten ist dessen Umsetzung mit Alkoholen, beispielsweise Methanol oder Ethanol, zu den entsprechenden tertiären Butylethern. Der Vorteil dieser Umsetzung besteht darin, dass das Isobuten in Gegenwart von linearen Butenen mit hoher Selektivität nahezu vollständig umgesetzt werden kann. Hierzu wurden diverse verfahrenstechnische Varianten für die Umsetzung mit Methanol zum MTBE entwickelt. Die Verwendung von MTBE, als Octanzahlverbesserer in Ottokraftstoffen wird zunehmend kritisch gesehen. Es besteht deshalb ein Interesse für die Abtrennung von Isobuten aus Kohlenwasserstoffströmen Methoden zu entwickeln, die den Anfall von MTBE vermeiden.

In EP 0 048 893 wird ein Verfahren zur gleichzeitigen Herstellung von Isobutenoligomeren und Alkyl-tert.-butylether (ATBE) aus C₄-Schnitten in einem Reaktor dargelegt. Als Katalysator wird ein saures Ionenaustauscherharz, das teilweise mit Metallen der siebten und achten Nebengruppe des Periodensystems der Elemente in elementarer Form (Oxidationsstufe 0) belegt ist, verwendet. Die Produkte und die nicht umgesetzten C₄-Kohlenwasserstoffe werden destillativ getrennt. Bei diesem Verfahren gehen circa 8 % der linearen Butene durch Oligomerisierung verloren. Der Verlust an 1-Buten liegt bei 7 %. Der Hauptnachteil dieses Verfahren ist jedoch, dass kein vollständiger Isobutenumsatz erreicht wird, so dass der Isobutengehalt in der abgetrennten C₄-Kohlenwasserstoff-Fraktion zu hoch ist, um daraus ein spezifikationsgerechtes 1-Buten zu gewinnen.

In DE 25 21 964 wird ein zweistufiges Verfahren zur Herstellung von Alkyl-tert.-butylethern (ATBE) beschrieben, bei dem in einer ersten Stufe Isobuten mit Alkohol umgesetzt wird, der entstandene Ether abgetrennt wird und der verbleibende Rest in eine zweite Reaktionsstufe geführt wird.

In US 6,472,568 wird eine zweistufige ETBE-Synthese beschrieben, bei der zumindest eine Stufe eine Reaktivdestillationskolonne ist und bei der im Zulauf zur ersten Reaktionsstufe ein molares Verhältnis von Ethanol zu Isobuten von 1,05 und im Zulauf der zweiten Stufe ein molares Verhältnis von 1,4 bis 4,0 vorliegt.

In RU 2167143 wird ein zweistufiges Verfahren zur Herstellung von ETBE beschrieben, bei dem am Eingang zur ersten Stufe ein molares Ethanol zu Isobuten Verhältnis von 0,86 zu 1 und am Eingang zum ersten Reaktor der zweiten Stufe ein molares Verhältnis von Ethanol zu Isobuten von 5 zu 1 eingestellt wird. Der dem Verfahren zugeführte Kohlenwasserstoffstrom weist 45 % Isobuten und 55 % sonstige Kohlenwasserstoffe auf. Erhalten wird ETBE und ein Kohlenwasserstoffstrom, der 1,4 % Isobuten aufweist und der damit, wenn es sich um 1-Buten handeln würde, nicht der oben genannten Spezifikation genügen würde.

In RU 2168490 wird ein zweistufiges Verfahren zur Herstellung von ETBE beschrieben, bei dem am Eingang zur ersten Stufe ein molares Ethanol zu Isobuten Verhältnis von mindestens 0,8 zu 1 und am Eingang zur zweiten Stufe ein molares Verhältnis von Ethanol zu Isobuten von 1,5 zu 1 bis 2 zu 1 eingestellt wird. Die zweite Stufe ist als Reaktivdestillation ausgeführt. Der dem Verfahren zugeführte Strom weist einen Isobutengehalt von 10 % auf. Der erhaltene Kohlenwasserstoffstrom weist 2,1 % Isobuten auf und würde damit, wenn es sich,um 1-Buten handeln würde, nicht der oben genannten Spezifikation genügen.

EP 1 199 296 A1 betrifft ein Verfahren zur Herstellung von hochreinem Raffinat II und Methyl-tert.-butylether, wobei das Isobuten eines isobutenhaltigen C4-Kohlenwasserstoffstroms in zwei Stufen mit Methanol umgesetzt wird.

WO 2004/065338 A betrifft ein Verfahren zur gekoppelten Herstellung von Butenoligomeren und tertiären Butylethem.

In den meisten der bekannten Verfahren wird nach der ersten Stufe zunächst der erhaltene Ether abgetrennt und der verbleibende Kohlenwasserstoffstrom in eine zweite Stufe überführt. Diese zweistufige Verfahrensweise ist relativ aufwändig. Zudem weisen die Verfahren den Nachteil auf, dass das erhaltene 1-Buten einen Anteil an Isobuten von deutlich mehr als 2000 wppm aufweist.

Ausgehend von diesem Stand der Technik bestand die Aufgabe der vorliegenden Erfindung in der Bereitstellung eines einfachen Verfahrens zur Herstellung von weniger als 2000 wppm Isobuten (bezogen auf das 1-Buten) aufweisendem 1-Buten bzw. 1-Buten aufweisenden Fraktionen ausgehend von 1-Buten-haltigen Ausgangsgemischen, die bereits einen relativ geringen Gehalt an Isobuten von kleiner 8 Massen-% aufweisen.

### Beschreibung der Erfindung

Überraschenderweise wurde nun gefunden, dass die Herstellung von weniger als 2000 ppm Isobuten bezogen auf das 1-Buten aufweisendem 1-Buten aus technischen Mischungen von C₄-Kohlenwasserstoffen I, die mindestens 1-Buten, und von 2000 wppm bis 8 Massen-% Isobuten bezogen auf das 1-Buten sowie gegebenenfalls n-Butan, Isobutan und/oder 2-Butene enthalten, dadurch einfach möglich ist, dass zunächst zumindest ein Teil des in der technischen. Mischung I enthaltenen Isobutens mit Ethanol in einer Serie von zumindest einer Reaktionszone, bevorzugt zumindest zwei hintereinandergeschalteten Reaktionszonen, die. z. B. als einzelne Festbetten oder einzelne Reaktoren ausgeführt sein können, in Gegenwart eines sauren Katalysators zu Ethyl-tert.-butyl-ether (ETBE) umgesetzt wird, anschließend der Austrag II der letzten Reaktionszone der Serie in eine Destillation überführt wird, in der als Sumpfprodukt ein Ethyl-tert.-butylether aufweisender Strom III erhalten wird und in der als Kopfprodukt ein Strom IV erhalten, wird, der 1-Buten, Ethanol und gegebenenfalls andere Kohlenwasserstoffe wie n-Butan, Isobutan und/oder 2-Butene aufweist, darauffolgend das Ethanol aus dem Strom IV z.B. in einer Extraktion unter Erhalt einer an Ethanol abgereicherten, 1-Buten aufweisenden Fraktion abgetrennt wird, und gegebenenfalls die in der Extraktion erhaltene, an Ethanol abgereicherte Fraktion V in einer Destillation in eine Fraktion VI, die im Wesentlichen 1-Buten und gegebenenfalls Isobutan aufweist, und in mindestens einen weiteren Strom VII mit den anderen Kohlenwasserstoffen aufgetrennt wird, besonders einfach möglich ist, wenn die Umsetzung im ersten Reaktor der Serie mit einem zumindest dreifachen molaren Überschuss an Ethanol in Bezug auf das in Mischung I enthaltene Isobuten durchgeführt wird.

Gegenstand der vorliegenden Erfindung ist deshalb ein

Verfahren zur Herstellung einer 1-Buten-haltigen Fraktion, umfassend:
- über 99,6 Massen-% 1-Buten,
- weniger als 0,2 Massen-% Isobuten,
- weniger als 0,05 Massen-% 2-Butene,
aus technischen Mischungen von C₄-Kohlenwasserstoffen I, die mindestens 1-Buten und von 2000 wppm bis 8 Massen-% Isobuten bezogen auf den Gehalt an 1-Buten enthalten, gekennzeichnet durch die Verfahrensschritte
a) Umsetzung zumindest eines Teils des in der technischen Mischung I enthaltenen Isobutens mit Ethanol in einer Reaktionszone oder einer Serie von zumindest zwei hintereinandergeschalteten Reaktionszonen in Gegenwart eines sauren Katalysators zu Ethyl-tert.-butylether,
b) Überführung des Reaktoraustrags II des letzten Reaktors der Serie in eine thermische Abtrennung, in der eine Ethyl-tert.-butylether aufweisende Fraktion III und eine Fraktion IV erhalten wird, die 1-Buten und Ethanol aufweist und
c) Abtrennung des Ethanols aus der Fraktion IV unter Erhalt eines 1-Buten-haltigen Fraktion V, und optional
d) Abtrennung von gegebenenfalls in der Fraktion IV enthaltenen C₄-Kohlenwasserstoffen, die nicht 1-Buten oder Isobuten sind, in zumindest
einem weiteren Trennschritt unter Erhalt einer 1-Buten-haltigen Fraktion VI wobei die Umsetzung in der ersten Reaktionszone in Schritt a) mit einem zumindest dreifachen molaren Überschuss an Ethanol in Bezug auf das in Mischung I enthaltene Isobuten durchgeführt wird.

Das erfindüngsgemäße Verfahren hat den Vorteil, dass 1-Buten-haltige Einsatzstoffströme mit geringen Konzentrationen an Isobuten auf relativ einfache Weise zu 1-Buten bzw. 1-Buten-haltigen Fraktionen bzw. Gemischen verarbeitet werden können, die weniger als 2000 wppm an Isobuten aufweisen. Das erfindungsgemäße Verfahren ist insbesondere deshalb einfacher als herkömmliche Verfahren, weil es keinen destillativen Trennschritt zwischen den Reaktionsschritten aufweist, bei dem ETBE aus dem Reaktionsgemisch abgetrennt wird. Die Verwendung von Ethanol hat den Vorteil, dass in dem erfindungsgemäßen Verfahren z. B. Bioethanol eingesetzt werden kann, so dass hochreines 1-Buten erhältlich ist, ohne dass zur Entfernung des Isobutens Methanol, welches häufig aus fossilen Brennstoffen hergestellt wird, eingesetzt werden muss.

Das als Nebenprodukt anfallende ETBE kann allein, oder gemeinsam mit dem eingesetzten überschüssigen Ethanol als Kraftstoffzusatz verwendet werden. ETBE zeichnet sich im Vergleich mit MTBE durch eine bessere Umweltverträglichkeit aus.

Ein weiterer Vorteil des erfindungsgemäßen Verfahren liegt darin, dass bei der Umsetzung von Isobuten mit Ethanol kaum 1-Buten zu 2-Butenen isomerisiert wird. Dies erhöht die Ausbeute an 1-Buten. Wenn das Edukt weder n-Butan oder Isobutan noch 2-Butene enthält, insbesondere wenn das Edukt ausschließlich Isobuten, 1-Buten und Verbindungen, die bei höheren Temperaturen als 1-Buten sieden, aufweist, kann auf einen Verfahrensschritt d) verzichtet werden.

Nachfolgend wird das erfindungsgemäße Verfahren beispielhaft beschrieben.

Auch die Ansprüche selbst gehören zum Offenbarungsgehalt der vorliegenden Erfindung. Sind im nachfolgenden Text Bereiche bzw. Vorzugsbereiche angegeben, so sollen auch alle in diesen Bereichen liegenden, theoretisch möglichen Teilbereiche zum Offenbarungsgehalt der vorliegenden Erfindung gehören, ohne dass diese aus Gründen der besseren Übersichtlichkeit explizit genannt worden sind.

Das Verfahren zur Herstellung von weniger als 2000 ppm Isobuten bezogen auf das 1-Buten aufweisenden 1-Buten-haltigen Fraktionen aus technischen Mischungen von C₄-Kohlenwasserstoffen I, die mindestens 1-Buten und von 2000 wppm bis 8 Massen-% Isobuten, vorzugsweise von 2500 wppm bis 3 Massen-% Isobuten, bezigen auf den Gehalt an 1-Buten enthalten, zeichnet sich durch aus, dass es die Verfahrensschritte.
a) Umsetzung zumindest eines Teils des in der technischen Mischung I enthaltenen Isobutens vorzugsweise das gesamte in der technischen Mischung I enthaltene Isobuten mit Ethanol in einer Reaktionszone oder einer Serie von zumindest zwei hintereinandergeschalteten Reaktionszonen in Gegenwart eines sauren Katalysators zu Ethyl-tert.-butylether,
b) Überführung des Reaktoraustrags II des letzten Reaktors/der letzten Reaktionszone der Serie in eine thermische Abtrennung, in der eine Ethyl-tert.-butylether aufweisende Fraktion III und eine Fraktion IV erhalten wird, die 1-Buten und Ethanol aufweist und
c) Abtrennung des Ethanols aus der Fraktion IV unter Erhalt eines 1-Buten-haltigen Fraktion V, und optional
d) Abtrennung von gegebenenfalls in der Fraktion IV enthaltenen C₄-Kohlenwasserstoffen, die nicht 1-Buten oder Isobuten sind, in zumindest einem weiteren Trennschritt unter Erhalt einer 1-Buten-haltigen Fraktion VI
aufweist, wobei die Umsetzung in der ersten Reaktionszone in Schritt a) mit einem zumindest dreifachen molaren Überschuss, vorzugsweise mit einem 3- bis 25-fachen molaren Überschuss, bevorzugt mit einem 5- bis 20-fachen molaren Überschuss und besonders bevorzugt mit einem 10-bis 15-fachen Überschuss an Ethanol in Bezug auf das in Mischung I enthaltene Isobuten durchgeführt wird. Durch diese Maßnahme kann insbesondere erreicht werden, dass vorzugsweise weniger als 1 %, bevorzugt weniger als 0,2 % und besonders bevorzugt weniger als 0,05 % des 1-Butens zu 2-Butenen isomerisiert wird. Das Verhältnis von Isobuten zu Ethanol bei der Umsetzung in der ersten Reaktionszone in Verfahrensschritt a) bezieht sich auf die Eingangskonzentrationen dieser beiden Edukte.

Weist die eingesetzte technische Mischung von C₄-Kohlenwasserstoffen I neben 1-Buten und Isobuten noch weitere Kohlenwasserstoffe, insbesondere Isobutan, n-Butan und/oder 2-Butene auf, so kann die in Verfahrensschritt c) erhaltene, an Ethanol abgereicherte, 1-Buten-haltige Fraktion V in dem optionalen Verfahrensschritt d) in eine 1-Buten-haltige Fraktion VI, die im Wesentlichen 1-Buten aufweist, und in mindestens einen weiteren Strom der zumindest eine Verbindung, ausgewählt aus Isobutan, n-Butan und 2-Butenen aufweist, aufgetrennt werden.

### Veretherung gemäß Schritt a)

In dem erfindungsgemäßen Verfahren kann die sauer katalysierte Veretherung in Stufe a) so durchgeführt werden, dass zumindest eine Reaktionszone als Reaktivdestillation ausgeführt wird. Wenn die sauer katalysierte Veretherung in Stufe a) so durchgeführt wird, dass die letzte Reaktionszone als Reaktivdestillation ausgeführt ist, wird in dieser auch die Destillation gemäß Schritt b) durchgeführt. Auf einen separaten Schritt b) kann in diesem Fall verzichtet werden. Wenn möglichst wenig 1-Buten durch Isomerisierung zu den beiden 2-Butenen verloren gehen soll, kann es vorteilhaft sein, die Veretherung gemäß Stufe a) ohne die Verwendung einer Reaktivdestillationskolonne durchzuführen.

Die Veretherung des Isobutens wird als sauer katalysierte Reaktion durchgeführt. Als Ethanol kann hochreines Ethanol, reines Ethanol oder Ethanol verwendet werden, das geringe Mengen an Verunreinigungen aufweist. Bevorzugt liegt die Reinheit des eingesetzten Ethanols, angegeben in Massen-% an Ethanol, über 90%, besonders bevorzugt über 98 %. Der Gehalt an Wasser liegt bevorzugt unter 3 Massen-%, besonders bevorzugt unter 1 Massen-%, ganz besonders bevorzugt unter 0,5 Massen-%. Es kann Ethanol eingesetzt werden, welches ein Vergällungsmittel aufweist. Bevorzugt wird als Ethanol ein Ethanol eingesetzt, welches als Vergällungsmittel ETBE aufweist. Besonders bevorzugt wird als Ethanol ein Ethanol eingesetzt, das ein Vergällungsmittel, vorzugsweise ETBE, in einer Konzentration zwischen 0 bis 5 Massen-%, vorzugsweise von 0,05 bis 1 Massen-% und bevorzugt von 0,01 bis 0,2 Massen-% aufweist. Durch die Verwendung von mit ETBE vergälltem Ethanol wird vermieden, dass Fremdstoffe in den Prozess eingetragen werden.

Für die Umsetzung von Isobuten mit Alkoholen, insbesondere mit Methanol zu Methyl-tert.-butylether wurden diverse Verfahrensvarianten entwickelt (vergleiche: Ullmann's Encyclopedia of Industrial Chemistry, Online Version, 2004, Wiley & Sons, Stichwort Methyl-tert-Butylether, und dort zitierte Literatur; Obenaus, Fritz; Droste, Wilhelm, Erdoel & Kohle, Erdgas, Petrochemie (1980), 33(6), 271-275; DE 2629769; DE 2853769). Prinzipiell sind alle bekannten Verfahren zur Umsetzung des Isobutens mit Ethanol geeignet als Verfahrensschritt a) im Rahmen der vorliegenden Erfindung eingesetzt zu werden.

Bevorzugt wird in Schritt a) die Umsetzung in flüssiger Phase an einem sauren Ionenaustauscherharz durchgeführt. Die Reaktionszonen können durch herkömmliche Reaktoren verwirklicht werden. Als Reaktoren, in denen der Alkohol mit dem Isobuten bis nahe an das thermodynamische Gleichgewicht umgesetzt wird, können herkömmliche Festbettreaktoren (Rohrbündelreaktoren, adiabatische Festbettreaktoren, Kreislaufreaktoren) eingesetzt werden. Sie können mit oder ohne partieller Rückführung, wobei gegebenenfalls der Rückführungsstrom gekühlt werden kann, betrieben werden. Besonders bevorzugt wird in Schritt a) ein Reaktorsystem eingesetzt, das zwei Reaktionszonen, vorzugsweise zwei Reaktoren, insbesondere zwei Festbettreaktoren aufweist, bei denen der erste der beiden Reaktoren der Serie als Kreislaufreaktor optional mit externer Kühlung und der zweite Reaktor im geraden Durchgang, vorzugsweise bei gegenüber dem ersten Reaktor verringerter Temperatur betrieben wird.

Die Veretherung in Schritt a) kann bei Temperaturen von 10 bis 160 °C, bevorzugt bei Temperaturen von 20 bis 110 °C und besonders bevorzugt bei Temperaturen von 30 bis 70 °C durchgeführt werden. Der Druck, bei dem die Veretherung durchgeführt wird, beträgt vorzugsweise 5 bis 50 bar_{absolut} (bara), bevorzugt 10 bis 20 bara. Da das thermodynamische Gleichgewicht zwischen Ethanol/Isobuten und Ether bei tiefer Temperatur überwiegend auf der Seite des Ethers liegt, ist es bei Einsatz mehrerer Reaktionszonen bevorzugt, die erste der Reaktionszonen bei höherer Temperatur (hohe Reaktionsgeschwindigkeit) als die folgenden Reaktionszonen (Ausnutzung der Gleichgewichtslage) zu betreiben.

Als sauerer Katalysator wird in den Reaktionszonen, unabhängig davon ob diese z. B. als Rohrreaktoren oder als Reaktivdestillationskolonnen verwirklicht sind, vorzugsweise ein fester Stoff, der weder im Einsatzstoffgemisch noch im Produktgemisch löslich ist, mit sauren Zentren an seiner Oberfläche eingesetzt. Der Katalysator sollte unter Reaktionsbedingungen vorzugsweise keine sauren Stoffe an das Produktgemisch abgeben, weil dies zu Korrosion und Ausbeuteverlusten führen kann.

Die Aktivität der Katalysatoren wird vorzugsweise so gewählt, dass sie unter Reaktionsbedingungen die Addition von Ethanol an Isobuten katalysieren, jedoch kaum die Addition an lineare Butene. Weiterhin sollten die Katalysatoren die Oligomerisierung von linearen Butenen und die Diethyletherbildung aus zwei Molekülen eingesetzten Ethanols möglichst nicht oder nur wenig katalysieren. Im Hinblick auf eine hohe Ausbeute an 1-Buten und einen geringen Destillationsaufwand sollte die Aktivität für die Isomerisierung von 1-Buten zu 2-Buten vorzugsweise gering sein.

Als feste Katalysatoren können beispielsweise Zeolithe, säureaktivierte Bentonite und/oder Tonerden, sulfatierte Zirkoniumoxide, Montmorillonite oder saure Ionenaustauscherharze verwendet werden.

Eine im erfindungsgemäßen Verfahren in Verfahrenschritt a) bevorzugte Gruppe von eingesetzten sauren Katalysatoren sind feste Ionenaustauscherharze, insbesondere solche mit Sulfonsäuregruppen. Geeignete Ionenaustauscherharze sind beispielsweise solche, die durch Sulfonierung von Phenol/Aldehyd-Kondensaten oder von Cooligomeren von aromatischen Vinylverbindungen hergestellt werden. Beispiele für aromatische Vinylverbindungen zur Herstellung der Cooligomere sind: Styrol, Vinyltoluol, Vinylnaphthalin, Vinylethylbenzol, Methylstyrol, Vinylchlorbenzol, Vinylxylol und Divinylbenzol. Insbesondere werden die Cooligomeren, die durch Umsetzung von Styrol mit Divinylbenzol entstehen, als Vorstufe für die Herstellung von Ionenaustauscherharzen mit Sulfonsäuregruppen verwendet. Die Harze können gelförmig, makroporös oder schwammförmig hergestellt werden.

Die Eigenschaften dieser Harze, insbesondere spezifische Oberfläche, Porosität, Stabilität, Quellung bzw. Schrumpfung und Austauschkapazität, können durch den Herstellprozess variiert werden.

Im erfindungsgemäßen Verfahren können die Ionenaustauscherharze in ihrer H-Form eingesetzt werden. Stark saure Harze des Styrol-Divinylbenzol-Typs werden u. a. unter folgenden Handelsnamen verkauft: Duolite^{®} C20, Duolite^{®} C26, Amberlyst^{®} 15, Amberlyst^{®} 35, Amberlyst^{®} 46, Amberlite^{®} IR-120, Amberlite^{®} 200, Dowex^{®} 50, Lewatit^{®} SPC 118, Lewatit^{®} SPC 108, K2611, K2621, OC 1501.

Das Porenvolumen beträgt vorzugsweise von 0,3 bis 0,9 ml/g, insbesondere von 0,5 bis 0,9 ml/g. Die Korngröße des Harzes beträgt bevorzugt von 0,3 mm bis 1,5 mm, insbesondere von 0,5 mm bis 1,0 mm. Die Korngrößenverteilung kann enger oder weiter gewählt werden. So können beispielsweise Ionenaustauscherharze mit sehr einheitlicher Korngröße (monodisperse Harze) eingesetzt werden. Die Kapazität des Ionenaustauscher beträgt, bezogen auf die Lieferform, vorzugsweise von 0,7 bis 2,0 eq/1, insbesondere von 1,1 bis 2,0 eq/1, bzw. vorzugsweise von 0,5 bis 5,5 mol/kg, insbesondere von 0,8 bis 5,5 mol/kg (Die Angaben zur Kapazität in mol/kg beziehen sich auf das jeweils bis zur Gewichtskonstanz im warmen Stickstoffstrom bei z. B. 105 °C getrocknete Ionentauscherharz.).

Im Reaktionsteil einer gegebenenfalls in Verfahrensschritt a) als Reaktionszone vorhandenen Reaktivdestillation können die gleichen Katalysatoren eingesetzt werden, wie sie in den einfachen Reaktoren eingesetzt werden können. In der Reaktivdestillationskolonne kann der Katalysator entweder in der Packung integriert sein, beispielsweise KataMax^{®} (wie in EP 0 428 265 beschrieben) oder KataPak^{®} (wie in EP 0 396 650 oder DE 298 07 007.3 U 1 beschrieben) oder auf Formkörpern aufpolymerisiert sein (wie in US 5 244 929 beschrieben).

Es kann vorteilhaft sein, wenn die Umsetzung in Schritt a) bei einer Temperatur in zumindest einer Reaktionszone von 30 bis 50 °C durchgeführt wird und ein Katalysator eingesetzt wird, der eine Kapazität von größer 3 bis 5,5 mol/kg aufweist. Katalysatoren, die eine Kapazität im angegebenen Bereich aufweisen, sind z. B. die Ionenaustauscherharze "Amberlyst^{®} 15" und "Amberlyst^{®} 35" der Firma Rohm und Haas. Ebenso kann es vorteilhaft sein, wenn die Umsetzung in Schritt a) bei einer Temperatur in zumindest einer Reaktionszone von 51 bis 70 °C durchgeführt wird und ein Katalysator eingesetzt wird, der eine Kapazität von 0,5 bis 3 mol/kg aufweist. Ein Katalysator, der eine Kapazität im angegebenen Bereich aufweist, ist z. B. das Ionenaustauscherharz "Amberlyst^{®} 46" der Firma Rohm und Haas. Durch die genannte Anpassung der Reaktionstemperatur an die Kapazität des Katalysators kann die Entstehung von 2-Butenen durch eine Isomerisierung von 1-Buten zu 2-Buten verringert bzw. verhindert werden. Die Angaben zur Kapazität beziehen sich auf das jeweils bis zur Gewichtskonstanz im warmen Stickstoffstrom (z. B. 105 °C) getrocknete Ionentauscherharz.

Besonders bevorzugt wird die Umsetzung im ersten Verfahrensschritt vor dem Destillationsschritt (vor der Destillationskolonne oder vor der Reaktivdestillationskolonne) mit einer LHSV (Liquid Hourly Space Velocity) von 0,3 bis 2,5 m³/(m³_{KAT}h), vorzugsweise von 0,5 bis 2 m³/(m³_{KAT}h) durchgeführt (Volumen Edukt zu Volumen Katalysator je Stunde). Ganz besonders bevorzugt werden diese Verfahrensparameter dann eingestellt, wenn auch die in den vorangegangenen Abschnitten als bevorzugt angeführten Bedingungen bezüglich Temperatur, Druck und/oder Katalysatorkapazität verwendet werden.

Wie bereits angeführt, kann in einer Ausführungsform des erfindungsgemäßen Verfahrens die Addition des Ethanols an das Isobuten in Gegenwart eines sauren Katalysators gemäß Schritt a) so durchgeführt werden, dass zumindest eine Reaktionszone als Reaktivdestillation ausgeführt wird. So kann die sauer katalysierte Veretherung in Schritt a) insbesondere in mindestens drei Reaktionszonen durchgeführt werden, wobei zumindest eine, besonders bevorzugt die letzte Reaktionszone als Reaktivdestillation ausgeführt wird. In der/den z. B. als Festbettreaktor/en ausgeführten Reaktionszone/n wird dabei zunächst an einem sauren Katalysator aus der Isobuten-haltigen Fraktion I und dem Ethanol ein Reaktionsgemisch hergestellt, das hinsichtlich seiner Isobuten-, Ethanol- und tert.-Butylether-Konzentration in der Nähe des thermodynamischen Gleichgewichts liegt. Der Umsatz des Isobutens beträgt dabei bevorzugt mehr als 80 %. Dieses Gemisch wird in der nächsten/letzten Reaktionszone in die Reaktivdestillationskolonne eingespeist, wo ein weiterer Teil des Isobutens zum Ether umgesetzt wird. Ganz besonders bevorzugt wird der Schritt a) in einem Reaktorsystem durchgeführt, das zwei in Reihe geschaltete Reaktoren und eine Reaktivdestillation aufweist, wobei der erste der beiden Reaktoren bevorzugt als Reaktor mit Rückführung eines Teils des Reaktionsproduktes und der zweite Reaktor im geraden Durchgang betrieben wird, und der Austrag aus dem zweiten Reaktor in die Reaktivdestillation eingespeist wird.

Im Reaktionsteil der Reaktivdestillation können die gleichen Katalysatoren, wie die oben für die einfache Ausführungsform der Verfahrensstufe ohne die Verwendung einer Reaktivdestillation beschriebenen, eingesetzt werden.

Die Umsetzung des Isobutens mit Ethanol zu ETBE erfolgt in der Reaktivdestillation vorzugsweise im Temperaturbereich von 10 bis 140 °C, bevorzugt bei 30 bis 90 °C, besonders bevorzugt bei 40 bis 70 °C (Temperatur im Bereich der Kolonne, in der sich der Katalysator befindet. Die Sumpftemperatur der Kolonne kann deutlich höher liegen).

Insbesondere wird das ETBE durch Umsetzung mit Ethanol in einer Weise hergestellt, wie es in DE 101 02 082 für die Reaktion von Methanol mit Isobuten zu MTBE beschrieben wird. Das Isobuten aufweisende C₄-Kohlenwasserstoffgemisch wird zusammen mit Ethanol in den/die Vorreaktor(en) eingespeist. In den Vorreaktoren entsteht ein Gemisch, in dem Isobuten, Ethanol und ETBE im Gleichgewicht oder nahezu im Gleichgewicht stehen. Dieses Reaktionsgemisch wird in die Reaktivdestillationskolonne eingeleitet.

Im Zulauf der Reaktivdestillationskolonne ist mehr Ethanol enthalten, als für die vollständige Umsetzung des noch vorhandenen Isobutens gebraucht wird. Der Alkoholüberschuss sollte zudem derart bemessen werden, dass eine ausreichende Alkoholmenge für das sich bildende Azeotrop aus Ethanol und C₄-Kohlenwasserstoffen vorhanden ist.

Optional kann, beispielsweise wenn der Ethanolgehalt im Kolonnenzulauf unter dem maximal zulässigen Wert liegt, zusätzlicher Ethanol zum Kolonnenzulauf zugemischt werden. Darüber hinaus kann am Kopf der Reaktivdestillationskolonne oberhalb des Kolonnenzulaufs unterhalb eines Flüssigkeitsverteilers oder in einem Flüssigkeitsverteiler oberhalb oder im Bereich der Reaktivzone, vorzugsweise im Bereich der Reaktivzone über eine gesonderte Einrichtung eine Ethanol-Einspeisung erfolgen. Eine zusätzliche Einspeisung von Ethanol kann z. B. in den Rücklauf der Kolonne oder direkt in die reaktiven Packungen erfolgen. Die zusätzliche Ethanolzugabe sollte so bemessen sein, dass in den Packungen der Reaktivzone der Ethanolgehalt in der Flüssigphase vorzugsweise größer-gleich 1,5 Massen-%, bevorzugt größer-gleich 2 Massen-% und besonders bevorzugt von 2 bis 3 Massen-% beträgt.

Bevorzugt weist die Reaktivdestillationskolonne oberhalb der Katalysatorpackung einen Bereich rein destillativer Trennung, vorzugsweise mit 5 bis 20, bevorzugt mit 5 bis 15 und besonders bevorzugt mit 7 bis 10 theoretische Trennstufen auf. Die Katalysatorzone lässt sich mit einer destillativen Wirkung von 1 bis 5 theoretischen Trennstufen pro Meter Packungshöhe abschätzen. Die Trennzone unterhalb des Katalysators kann vorzugsweise von 12 bis 36, insbesondere von 15 bis 25 theoretische Trennstufen umfassen. Die Höhe der Katalysatorzone/Reaktivzone lässt sich in Abhängigkeit vom gewünschten Isobuten-Umsatz durch einfache Vorversuche ermitteln. Die Katalysatormenge wird vorzugsweise so groß gewählt, dass der gewünschte Restisobutengehalt in Bezug auf das 1-Buten im Kopfprodukt erreicht wird.

Der Zulauf zur Reaktivdestillationskolonne kann oberhalb oder unterhalb der Katalysatorzone erfolgen. Der Zulauf zur Reaktivdestillationskolonne erfolgt vorzugsweise unterhalb der reaktiven Packung, bevorzugt 3 bis 13, besonders bevorzugt 4 bis 10 theoretische Trennstufen unterhalb der reaktiven Packung.

Die Reaktivdestillationskolonne wird vorzugsweise bei Drücken, gemessen am Kolonnenkopf, von 3 bara bis 25 bara, bevorzugt 5 bara bis 15 bara betrieben, insbesondere von 5 bara bis 9 bara. Die hydraulische Belastung in der katalytischen Packung der Kolonne beträgt bevorzugt 10 % bis 110 %, vorzugsweise 20 % bis 90 % und besonders bevorzugt 35 bis 75 % ihrer Flutpunktbelastung. Unter hydraulischer Belastung einer Destillationskolonne wird die gleichmäßige strömungstechnische Beanspruchung des Kolonnenquerschnitts durch den aufsteigenden Dampf-Massenstrom und den rücklaufenden Flüssigkeits-Massenstrom verstanden. Die obere Belastungsgrenze kennzeichnet die maximale Belastung durch Dampf und Rücklaufflüssigkeit, oberhalb derer die Trennwirkung infolge Mitreißens oder Stauens der Rücklaufflüssigkeit durch den aufsteigenden Dampfstrom absinkt. Die untere Belastungsgrenze kennzeichnet die minimale Belastung, unterhalb derer die Trennwirkung absinkt oder zusammenbricht infolge unregelmäßiger Strömung- z. B. durch Entnetzen der Packung oder starkes Durchregnen der Böden. (Vauck/Müller, "Grundoperationen chemischer Verfahrenstechnik", S. 626, VEB Deutscher Verlag für Grundstoffindustrie.)

Am Flutpunkt werden die vom Gas an die Flüssigkeit übertragenen Schubspannungen so groß, dass die gesamte Flüssigkeitsmenge in Form von Tropfen mit dem Gas mitgerissen wird oder dass es zu Phaseninversion in der Kolonne kommt (J. Mackowiak, "Fluiddynamik von Kolonnen mit modernen Füllkörpern und Packungen für Gas/Flüssigkeitssysteme", Otto Salle Verlag 1991).

Die Reaktivdestillationskolonne wird vorzugsweise mit Rücklaufverhältnissen von 0,2 bis 4 betrieben, insbesondere mit solchen, die von 0,4 bis 2, bevorzugt von 0,5 bis 1 betragen.

Wird in der Stufe a) als letzte Reaktionszone eine Reaktivdestillationskolonne eingesetzt, so kann wie bereits beschrieben in dieser der Schritt b), nämlich die Abtrennung des ETBE von den nicht umgesetzten Kohlenwasserstoffen stattfinden. Auf einen separaten Schritt b) kann dann gegebenenfalls verzichtet werden.

Unter den Oberbegriff der Reaktivdestillation fallen alle verfahrenstechnischen Maßnahmen, bei denen Destillation und Reaktion gleichzeitig durchgeführt werden. In den beschriebenen Reaktoren wird dies durch eine besondere Ausführung der Packungen in einer Kolonne erreicht. Es ist im erfindungsgemäßen Verfahren aber auch möglich, diese Bereiche räumlich zu trennen, ohne auf die Vorteile einer Reaktivdestillation zu verzichten.

### Thermische Trennung gemäß Schritt b)

Die thermische Trennung des Austrags II aus dem Schritt a) kann in Schritt b) z. B. durch Destillation oder Fraktionierungen erfolgen. Vorzugsweise erfolgt die thermische Trennung durch Destillation, die auf übliche Weise durchgeführt werden kann. Die Destillation kann z. B. dadurch erfolgen, dass der Austrag II aus dem letzten Reaktor/der letzen Reaktionszone der Serie des Verfahrensschrittes a) in eine Destillationskolonne eingespeist wird. Die Kolonne kann mit einem Sumpfverdampfer und einem Kondensator für das Kopfprodukt ausgerüstet sein. Als Sumpfprodukt III der Destillationskolonne wird ein Strom erhalten, der ETBE und gegebenenfalls überschüssigen Alkohol enthält. Außerdem kann Strom III noch Diethylether enthalten. Das Kopfprodukt IV kann teilweise als Rücklauf in die Kolonne zurückgefahren werden. Der andere Teil, kann dem Verfahrensschritt c) zugeführt werden.

Eine eingesetzte Destillationskolonne weist vorzugsweise mehr als 20, bevorzugt mehr als 25, besonders bevorzugt von 30 bis 50 theoretische Trennstufen auf. Das Rücklaufverhältnis ist, in Abhängigkeit von der realisierten Stufenzahl vorzugsweise kleiner-gleich 1, und nimmt besonders bevorzugt Werte von 0,9 bis 0,6 ein. Die Kondensation kann gegen Kühlwasser oder Luft durchgeführt werden. Zur Beheizung des Verdampfers der Kolonne kann z. B. Dampf eingesetzt werden. Es kann vorteilhaft sein, dass der Zulaufstrom zur Kolonne zumindest teilweise vorverdampft in die Kolonne geleitet oder direkt in die Kolonne geflasht wird. Bevorzugt wird dafür dem Zulaufstrom in einem externen Wärmeübertrager Wärme, z. B. durch Nutzung von Abwärme, zugeführt. Zum Erzielen einer teilweisen Verdampfung ist ein Kettle-Verdampfer die bevorzugte Ausführungsform des Wärmeübertragers. Es kann außerdem vorteilhaft sein, wenn ein mit Prozess- oder Abwärme auf niedrigerem Temperaturniveau beheizter Zwischenverdampfer im unteren Teil der Kolonne eingesetzt wird.

Der Zulauf zu der Kolonne erfolgt in Verfahrensschritt b) vorzugsweise auf der 10 bis 15 theoretischen Trennstufe. Die Kolonne wird vorzugsweise mit einem Druck von 4 bis 11, bevorzugt von 5 bis 8 bara betrieben. Die Kopftemperatur der in Verfahrensschritt b) eingesetzten Kolonne beträgt vorzugsweise von 40 bis 70, bevorzugt von 45 bis 60 °C.

Beinhaltet der Verfahrensschritt a) eine Reaktivdestillation, so kann der Verfahrensschritt b) teilweise oder vollständig, vorzugsweise vollständig bereits bei der Durchführung der Reaktivdestillation stattfinden und ein separater Schritt b) kann gegebenenfalls entfallen.

### Abtrennung des Ethanols gemäß Schritt c)

Zur Abtrennung des Ethanols aus dem Strom IV können verschiedene dem Fachmann bekannte Methoden eingesetzt werden. So kann die Abtrennung des Ethanols z. B. durch Membranverfahren oder durch Extraktion erfolgen. Vorzugsweise erfolgt die Abtrennung von Ethanol aus dem Strom IV durch Extraktion. Für die Extraktion können alle Extraktionsmittel eingesetzt werden, die geeignet sind Ethanol aus dem Strom IV zu extrahieren. Vorzugsweise wird in Stufe c) eine Extraktion durchgeführt, in der als Extraktionsmittel Wasser oder eine wässrige Lösung eingesetzt wird.

Bevorzugt wird das Kopfprodukt aus dem Verfahrensschritt b), welches am Kopf der Destillationskolonne gemäß Schritt b) oder am Kopf der Reaktivdestillationskolonne (Schritte a) und b)) erhalten wird, in eine Extraktionskolonne überführt, in die im Gegenstrom ein Extraktionsmittel, wie z. B. Wasser, über einen in der Nähe des Kopfes befindlichen Zulauf eingespeist wird. Das Extraktionsmittel kann über den Ablauf am Sumpf der Kolonne entnommen werden. Am Kopf der Kolonne wird als Produkt der Extraktion ein Strom aus in Stufe a) nicht umgesetzten Kohlenwasserstoffen V erhalten, der an Ethanol abgereichert ist. Dieser wird dem erfindungsgemäßen Verfahrensschritt d) zugeführt, Das im Sumpf der Kolonne anfallende mit Ethanol angereicherte Extraktionsmittel kann destillativ aufgetrennt werden und das Ethanol gegebenenfalls, wenn als Extraktionsmittel Wasser eingesetzt wurde, bevorzugt nach einer Trocknung dem Prozess als Ausgangsstoff in den Schritt a) zurückgeführt werden. Die Trocknung kann mit Hilfe eines Schleppmittels destillativ, durch Adsorption mit Hilfe eines Adsorptionsmittels, wie z. B. Molsieb, oder mittels einer Membrananlage (z. B. Pervaporation, Umkehrosmose oder Permeation) erfolgen.

Der Verfahrensschritt c) kann vorzugsweise in einer Extraktionskolonne durchgeführt werden. Vorzugsweise weist die Extraktionskolonne von 5 bis 20, bevorzugt 10 bis 15 theoretische Trennstufen auf. Die Extraktion im Verfahrensschritt c) wird vorzugsweise bei einem Druck von 5 bis 12, bevorzugt von 7 bis 10 bara und vorzugsweise bei einer Temperatur von 30 bis 60 °C, besonders bevorzugt von 35 bis 45 °C durchgeführt. Dass Verhältnis von Extraktionsmittel, insbesondere Wasser zum Kopfprodukt aus dem Verfahrensschritt b) beträgt vorzugsweise von 0,05 bis 0,5, bevorzugt von 0,1 bis 0,25 und besonders bevorzugt von 0,15 bis 0,2.

### Destillation gemäß Schritt d)

Weist das Einsatzstoffgemisch neben 1-Buten und Isobuten weitere Stoffe, insbesondere n-Butan, Isobutan und/oder 2-Butene auf, so kann es vorteilhaft sein, die in Verfahrensschritt c) erhaltene, an Ethanol abgereicherte bzw. von Ethanol befreite, 1-Buten aufweisende Fraktion V in eine Fraktion VI die 1-Buten bzw. ein 1-Buten aufweisendes Gemisch aufweist, und in zumindest einen weiteren Strom der zumindest eine Verbindung, ausgewählt aus Isobutan, n-Butan und 2-Butenen aufweist, aufzutrennen. Das Auftrennen erfolgt vorzugsweise destillativ. Die Abtrennung des 1-Butens VI durch Destillation der Fraktion V kann in einer oder mehreren Destillationskolonnen erfolgen.

Sind in der eingesetzten technischen Mischung von C₄-Kohlenwasserstoffen n-Butan, Isobutan und 2-Butene vorhanden, so erfolgt die Abtrennung des 1-Butens in einer bevorzugten Ausführungsform des Verfahrensschritts d) so, dass die Abtrennung des 1-Butens in einer Destillationskolonne durchgeführt wird, in der als Kopfprodukt eine 1-Buten-Fraktion D-d1, die Isobutan enthält, und als Sumpfprodukt eine mindestens 2-Butene, n-Butan sowie nicht abgetrenntes 1-Buten enthaltenden Fraktion VII gewonnen wird.

Bevorzugt wird die Trennung in einer oder zwei Superfraktionierkolonnen durchgeführt. Der Zulaufboden der Kolonne oder Kolonnen wird für das jeweilige Zulaufgemisch nach der technisch üblichen Praxis so optimiert, dass die Trennaufgabe mit minimalem Energieaufwand erfüllt wird. Wegen der engen Siedelage der zu trennenden Gemische werden die Kolonnen mit vorzugsweise mehr als 100, bevorzugt mehr als 125, besonders bevorzugt mit 150 oder mehr und ganz besonders bevorzugt mit 150 bis 200 theoretischen Trennstufen ausgeführt. Das Rücklaufverhältnis (Rücklaufmenge zu Destillatabnahme) ist von der realisierten Stufenzahl und vom Betriebsdruck abhängig und kann für das Einsatzgemisch optimiert werden. Vorzugsweise beträgt das Rücklaufverhältnis kleiner-gleich 100, bevorzugt kleiner 75, besonders bevorzugt von 10 bis 60. Die Kondensation kann gegen Kühlwasser oder Luft durchgeführt werden. Der Destillatbehälter wird vorzugsweise als flüssig-flüssig-Abscheider ausgeführt. Dadurch kann gegebenenfalls im Zulaufstrom enthaltenes Wasser als zweite Phase im Destillatbehälter abgetrennt werden und es kann ein technisch wasserfreies Sumpfprodukt erhalten werden.

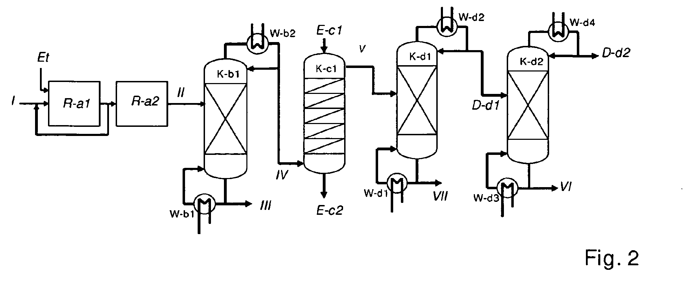

Zur Beheizung des Verdampfers der Kolonne kann ein üblicher Wärmeträger, wie z. B. Dampf oder Warmwasser sowie bevorzugt Abwärme aus anderen Prozessen eingesetzt werden. In letzterem Fall kann es vorteilhaft sein, die Kolonne mit mehr als einem Verdampfer auszustatten. Die Kolonne wird bevorzugt als einfache Kolonne mit mindestens einem Verdampfer und mindestens einem Kondensator ausgerüstet. Wegen des hohen Energiebedarfs und der kleinen Temperaturdifferenz zwischen Sumpf und Kopf der Kolonne sind Energiesparschaltungen besonders bevorzugte Ausführungsformen. Exemplarisch sei hier auf die Methode der Brüdenverdichtung verwiesen. Eine weitere besonders bevorzugte Schaltung ist die Zweidruckschaltung (double effect distillation) in Integration mit einer zweiten Kolonne. Die zweite Kolonne kann bevorzugt eine parallelgeschaltete Kolonne mit gleicher oder unterschiedlicher Trennaufgabe sein. Dabei wird eine der Kolonnen bei so hohem Druck gefahren, dass ihre Kondensationstemperatur zur Beheizung der anderen Kolonne ausreicht. Bei der wärmetechnischen Verschaltung von Kolonnen mit unterschiedlichen Trennaufgaben kann prinzipiell jede geeignete Kolonne aus dem erfindungsgemäßen Verfahren aber auch eine Kolonnen, die außerhalb des erfindungsgemäßen Verfahren am Anlagenstandort vorhanden ist, mit der erfindungsgemäßen Kolonne des Verfahrensschrittes d) verschaltet werden. Bei Ausführung des erfindungsgemäßen Verfahrens nach Fig. 2 bietet sich die Verschaltung der Kolonnen K-d1 und K-d2 in Form einer Zweidruckschaltung an.

Die Trennung gemäß Verfahrensschritt d) wird vorzugsweise bei einem Druck von 5 bis 11 bara, bevorzugt bei einem Druck von 6 bis 8 bara durchgeführt. Die Kopftemperatur bei welcher die Trennung durchgeführt wird beträgt vorzugsweise von 35 bis 65 °C, bevorzugt 45 bis 50 °C. Wenn eine Wärmeintegration vorgesehen wird, kann es vorteilhaft sein, wenn der Verfahrensschritt i) bei höherer Temperatur und/oder höherem Druck durchgeführt wird.

Das als Kopfprodukt erhaltene Gemisch D-d1, das 1-Buten und Isobutan enthält, kann in einer zweiten Kolonne, die im Wesentlichen genauso ausgeführt ist wie die erste Kolonne, in 1-Buten, welches als Sumpffraktion VI anfällt, und eine Isobutan-reiche Fraktion (Kopfprodukt) D-d2 aufgetrennt werden.

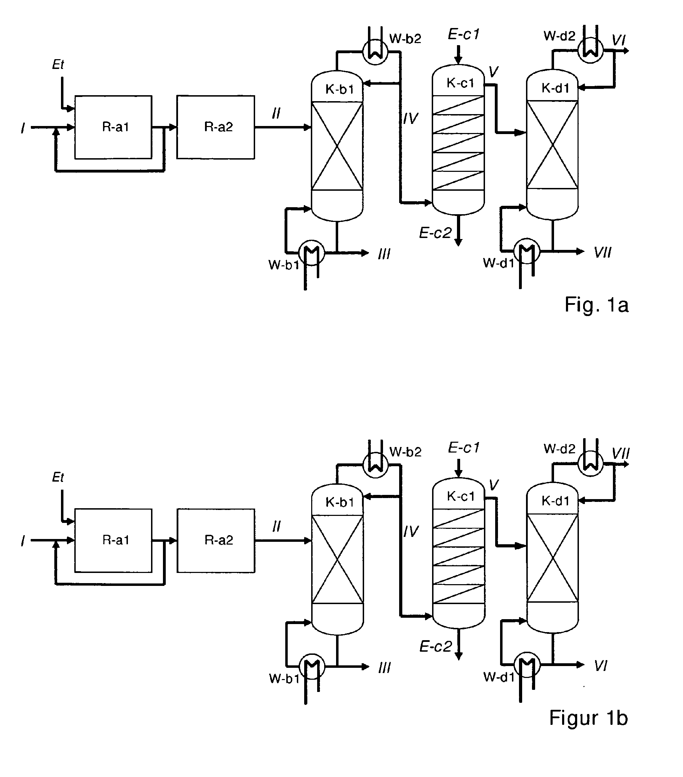

Wenn in der erfindungsgemäß eingesetzten technischen Mischung I kein Isobutan enthalten ist, kann gegebenenfalls auf die zweite Kolonne verzichtet werden. Eine solche Ausführungsform des erfindungsgemäßen Verfahrens, bei dem ein Verfahrensschritt d) vorhanden ist, der nur eine Destillationskolonne zur Trennung von 1-Buten von n-Butan und/oder 2-Butenen aufweist, ist in Fig. 1a dargestellt.

Wenn in der erfindungsgemäß eingesetzten technischen Mischung I kein n-Butan und/oder keine 2-Butene oder wenn in der technischen Mischung zumindest weniger als 1000 wppm n-Butan und/oder zumindest weniger als 100 wppm 2-Butene enthalten sind und im Schritt a) praktisch keine Isomerisierung von 1-Buten auftritt, kann gegebenenfalls auf die erste Kolonne verzichtet werden. Eine solche Ausführungsform des erfindungsgemäßen Verfahrens, bei dem ein Verfahrensschritt d) vorhanden ist, der nur eine Destillationskolonne zur Trennung von 1-Buten von Isobutan aufweist, ist in Fig. 1b dargestellt.

In Verfahrensschritt d) können, je nach Ausgangszusammensetzung der C₄-Kohlenwasserstoffe, neben dem 1-Buten Isobutan-reiche Fraktionen anfallen. Diese können weiter, vorzugsweise zu reinem Isobutan, aufgereinigt werden. Das bei der Aufarbeitung gewonnene Isobutan hat vorzugsweise eine Reinheit von mindestens 90 Massen-% Isobutan, besonders bevorzugt 95 Massen-% Isobutan und enthält bevorzugt weniger als 1000 wppm, besonders bevorzugt weniger als 200 wppm Olefine. Eine Aufreinigung zu reinem Isobutan kann beispielsweise durch vollständige Hydrierung der noch enthaltenen Alkene zu Alkanen und anschließende Destillation erfolgen.

Sollten in den Fraktionen V oder VI noch weitere Restmengen an Alkohol vorhanden sein, und sollen diese auf Grund der angestrebten Verwendung vorzugsweise entfernt werden, so kann dies z. B. dadurch erfolgen, dass der Alkohol in einem weiteren Extraktionsschritt mit Wasser ausgewaschen wird. Dieser Extraktionsschritt kann wie unter Schritt c) beschreiben durchgeführt werden. Es kann vorteilhaft sein, wenn nach einem weiteren Extraktionsschritt mit Wasser die erhaltene Fraktion in einem anschließenden Trocknungsschritt von Wasser befreit wird.

### Einsatzstoffe

In dem erfindungsgemäßen Verfahren können alle üblicherweise zur Verfügung stehenden technischen C₄-Kohlenwasserstoffgemische, die 1-Buten, n-Butan und 2000 wppm bis 8 Massen-% Isobuten bezogen auf das 1-Buten, vorzugsweise 2500 wppm bis 3 Massen-% Isobuten bezogen auf das 1-Buten aufweisen, eingesetzt werden. Geeignete Isobuten-haltige C₄-Ströme können beispielsweise solche sein, wie sie bei der Aufarbeitung von C₄-Strömen z. B. aus Raffinerien, aus Crackern (beispielsweise Steamcracker, Katcracker), aus Fischer-Tropsch-Synthesen, aus der Dehydrierung von Butanen, aus Skelettisomerisierung linearer Butene und solchen, erhalten durch Metathese von Olefinen, erhalten werden. Diese Techniken sind in der Fachliteratur beschrieben. (K.Weissermel, H. J. Arpe, Industrielle Organische Chemie, Wiley-VCH, 5. Auflage, 1998, Seiten 23 bis 24; 65 bis 99; 122 bis 124).

Bevorzugt werden C₄-Ströme eingesetzt, wie sie bei der Aufarbeitung von C₄-Strömen aus Katcrackern (FCC) oder aus Steamcrackern, die primär zur Produktion von Ethen und Propen betrieben werden und in denen als Rohstoffe beispielsweise Raffineriegase, Naphtha, Gasöl, LPG (liquified petroleum gas) und NGL (natural gas liquid) eingesetzt werden, gegebenenfalls nach Abtrennung eines Teils des Isobutens erhalten werden. Ganz besonders bevorzugt werden als Isobuten-haltige technische Mischungen Isobuten-arme Raffinat II-Fraktionen, beispielsweise aus TBA-, ETBE oder MTBE-Anlagen, Restströme aus Oligomerisierungen oder Polymerisierungen oder andere Isobuten-arme Rohstoffströme wie FCC-C₄ sowie beliebige Gemische aus den oben genannten Quellen eingesetzt.

Ganz besonders bevorzugt wird in dem erfindungsgemäßen Verfahren eine technische Mischung von C₄-Kohlenwasserstoffen I eingesetzt, die einen Anteil an 1-Buten von größer 50 Massen-%, vorzugsweise größer 70 Massen-%, bevorzugt größer 80 Massen-%, besonders bevorzugt größer 90 Massen-% und besonders bevorzugt größer 95 Massen-% aufweist. Solche technischen Mischungen können insbesondere aus herkömmlichen Verfahren zur Abtrennung von Isobuten aus 1-Buten-haltigen Strömen stammen.

Für das erfindungsgemäße Verfahren ist es vorteilhaft, mehrfach ungesättigte Kohlenwasserstoffe wie 1,3-Butadien aus dem Einsatzgemisch abzutrennen. Dies kann nach bekannten Verfahren, beispielsweise durch Extraktion, Extraktivdestillation oder Komplexbildung erfolgen (vgl. K.Weissermel, H.J. Arpe, Industrielle Organische Chemie, Wiley-VCH, 5. Auflage, 1998, Seite 119-121). Vorzugsweise werden in dem erfindungsgemäßen Verfahren technische Mischungen I eingesetzt, die einen Anteil an mehrfach ungesättigten Verbindungen von kleiner-gleich 20 Massen-ppm, bevorzugt kleiner-gleich 5 Massen-ppm aufweisen.

Eine Alternative zur Abtrennung der mehrfach ungesättigten Kohlenwasserstoffe ist eine selektive chemische Umsetzung. So kann beispielsweise 1,3-Butadien selektiv zu linearen Butenen hydriert werden, wie z. B. in EP 0 523 482 beschrieben. Auch durch selektive Umsetzungen des 1,3-Butadiens, zum Beispiel Dimerisierung zum Cyclooctadien, Trimerisierung zum Cyclododecatrien, Diels-Alder-Reaktion z. B. mit Maleinsäure oder Maleinsäureanhydrid, Polymerisations- oder Telomerisationsreaktionen, kann das 1,3-Butadien zumindest teilweise entfernt werden. Wurde ein Crack-C₄-Schnitt als Rohstoff eingesetzt, bleibt in allen Fällen ein Kohlenwasserstoffgemisch (z. B. Raffinat I oder selektiv hydriertes Crack-C₄ (HCC₄)) zurück, das hauptsächlich die gesättigten Kohlenwasserstoffe, n-Butan und Isobutan und die Olefine Isobuten, 1-Buten und 2-Butene enthält. Der Anteil an Isobuten in diesem Kohlenwasserstoffgemisch kann gegebenenfalls durch geeignete, wie oben beschriebene Verfahren auf einen erfindungsgemäßen Anteil von 2000 wppm bis 8 Massen-% bezogen auf den Gehalt an 1-Buten reduziert werden.

Bevorzugt wird in dem erfindungsgemäßen Verfahren in einer zusätzlichen Reinigungsstufe, die einer oder mehreren der Verfahrensschritte a), b), c) oder d) vorgeschaltet wird, in den C₄-Kohlenwasserstoffströmen enthaltene mehrfach ungesättigten Kohlenwasserstoffe selektiv katalytisch hydriert. Besonders bevorzugt ist zumindest vor dem Verfahrensschritt a) oder d) und ganz besonders bevorzugt vor der Verfahrensstufe d) eine solche Reinigungsstufe vorgesehen, insbesondere wenn nicht ausgeschlossen werden kann, dass die eingesetzten technischen C₄-Kohlenwasserstoffströme mehrfach ungesättigte Kohlenwasserstoffe aufweisen.

Bei den mehrfach ungesättigten Kohlenwasserstoffen handelt es sich hauptsächlich um 1,3-Butadien; 1,2-Butadien, Butenin und 1-Butin sind, wenn überhaupt, in deutlich geringerer Menge enthalten. Die Hydrierung kann in einem ein- oder mehrstufigen Hydrierprozess, vorzugsweise in einem mehrstufigen Hydrierprozess in der Flüssigphase an einem Palladiumkontakt erfolgen. Zur Absenkung des Gehalts an 1,3-Butadien unter vorzugsweise 1000 Massen-ppm (wppm), bevorzugt kleiner 100 wppm, besonders bevorzugt kleiner-gleich 20 wppm und ganz besonders bevorzugt kleiner-gleich 5 wppm wird dabei in der letzten Stufe der Hydrierung mit Zusatz eines Moderators gearbeitet, der die Selektivität des Palladiumkontakts erhöht. Bevorzugt wird als Moderator Kohlenmonoxid eingesetzt, das in einem Anteil von 0,05 bis 100 Massen-ppm (wppm) zugesetzt wird. Besonders bevorzugt erfolgt die Hydrierung der mehrfach ungesättigten Verbindungen in mindestens zwei Reaktionsstufen, wobei mindestens die letzte Reaktionsstufe in Anwesenheit von 0,05 bis 100 Massen-ppm Kohlenmonoxid durchgeführt wird. Der Gehalt an mehrfach ungesättigten Kohlenwasserstoffen sollte im Zulauf zu dieser Stufe unter 1 Massen-%, bevorzugt unter 0,5 Massen-%, betragen. In der Literatur ist diese Art der Selektivhydrierung von Restgehalten an 1,3-Butadien unter der Bezeichnung SHP (selective hydrogenation process) bekannt (vergleiche EP 0 081 041; Erdöl, Kohle, Erdgas, Petrochem. 1986, 39, 73).

Sind in den Isobuten-haltigen C₄-Strömen Mengen mehr als 1 Massen-% an mehrfach ungesättigten Kohlenwasserstoffen wie 1,3-Butadien enthalten, werden sie vorzugsweise in vorgeschalteten Hydrierungen umgesetzt. Diese Hydrierungen werden bevorzugt in der Flüssigphase an einem Palladiumkontakt durchgeführt. Je nach Gehalt an ungesättigten Kohlenwasserstoffen kann die Hydrierung in mehreren Stufen durchgeführt werden. Zur Umsetzung von Crack-C₄ aus einem Steam-Cracker mit einem Gehalt an 1,3-Butadien von typischerweise 38 bis 45 % hat sich eine zweistufige Ausführung der Hydrierung bewährt. Dabei können einzelne oder alle Stufen mit einer teilweisen Produktrückführung ausgestattet sein. Im Austrag sind so Konzentrationen an 1,3-Butadien kleiner 1 % erhältlich, so dass eine weitere Umsetzung in einer Selektivhydrierung (SHP) erfolgen kann.

Einsetzbare C₄-Kohlenwasserstoffgemische können vor dem Eintritt in das erfindungsgemäße Verfahren eine oder mehrere andere Prozessstufe(n) durchlaufen. Diese Prozessstufe(n) kann/können beispielsweise auch ein Verfahren bzw. Verfahrensschritt(e) zur Abtrennung von Isobuten aus C₄-Kohlenwasserstoffgemischen sein. Insbesondere können auch solche Gemische als Ausgangsgemisch in dem erfindungsgemäßen Schritt a) eingesetzt werden, wie sie bei der Herstellung von tert.-Butanol (TBA) aus Isobuten nach Abtrennung des TBA erhalten werden. Auf diese Weise kann jeweils ein individuell angepasstes Gesamtkonzept zur Aufarbeitung mit dem entsprechenden Produktportfolio realisiert werden.

Typische Verfahren, die den erfindungsgemäßen Verfahren vorgelagert sein können, sind Wasserwäschen, Reinigungsverfahren in Adsorbern, Trocknungsverfahren und Destillationen.

### Wasserwäsche

Durch eine Wasserwäsche können hydrophile Komponenten aus dem einzusetzenden technischen Kohlenwasserstoffgemisch, enthaltend Isobuten und lineare Butene, ganz oder teilweise entfernt werden, beispielsweise Stickstoffkomponenten. Beispiele für Stickstoffkomponenten sind Acetonitil oder N-Methylpyrrolidon (die z. B. aus einer 1,3-Butadien-Extraktivdestillation stammen können). Auch Sauerstoffverbindungen (z. B. Aceton aus FC-Cracker) können zum Teil über eine Wasserwäsche entfernt werden. Der Isobuten-haltige Kohlenwasserstoffstrom ist nach einer Wasserwäsche mit Wasser gesättigt. Um eine Zweiphasigkeit in den nachfolgenden Prozessschritten im Reaktor zu vermeiden, sollte dort die Reaktionstemperatur um ca. 10 °C über der Temperatur der Wasserwäsche liegen.

### Adsorber

Adsorber werden eingesetzt, um Verunreinigungen zu entfernen. Dies kann beispielsweise vorteilhaft sein, wenn in einem der Prozessschritte Edelmetallkatalysatoren zum Einsatz kommen. Oftmals werden Stickstoff oder Schwefelverbindungen über vorgeschaltete Adsorber entfernt. Beispiele für Adsorptionsmittel sind Aluminiumoxide, Molekularsiebe, Zeolithe, Aktivkohle, mit Metallen imprägnierte Tonerden. Adsorptionsmittel werden von diversen Firmen vertrieben, beispielsweise der Firma Alcoa (Selexsorb^{®}).

### Trocknung

Im Isobuten-haltigen Kohlenwasserstoffgemisch gegebenenfalls enthaltenes Wasser, das beispielsweise aus einer Wasserwäsche stammen kann, kann durch bekannte Verfahren zur Trocknung entfernt werden. Geeignete Verfahren sind beispielsweise die destillative Abtrennung des Wassers als Azeotrop. Dabei kann oftmals ein Azeotrop mit enthaltenen C₄-Kohlenwasserstoffen ausgenutzt werden oder es können Schleppmittel zugesetzt werden.

Die Trocknung des Kohlenwasserstoffgemisches kann aus diversen Gründen vorteilhaft sein, beispielsweise zur Verringerung der Bildung von Alkoholen (hauptsächlich tert.-Butylalkohol) in Verfahrensschritt a) oder zur Vermeidung von technischen Problemen durch Abscheidung von Wasser oder zur Vermeidung von Eisbildung bei niedrigen Temperaturen (z. B. bei der Zwischenlagerung).

### Destillation

Destillationsschritte können beispielsweise genutzt werden, um Verunreinigungen abzutrennen (beispielsweise Leichtsieder wie C₃-Kohlenwasserstoffe, Schwersieder wie C₅-Kohlenwasserstoffe) oder um Fraktionen mit unterschiedlichen Isobutenkonzentrationen zu erhalten. Dies kann sowohl direkt mit dem Raffinat I bzw. dem HCC₄ erfolgen oder nachdem eine oder mehrere andere Prozessstufe(n) durchlaufen wurden. Durch direkte Destillation des Raffinats I bzw. des HCC₄ ist beispielsweise eine Trennung in eine an 2-Butenen und n-Butan verarmte, Isobuten reichere Fraktion möglich.

Je nach Zusammensetzung des einzusetzenden technischen Kohlenwasserstoffgemisches und/oder nach den Reinheiten der Zielprodukte kann das technische Kohlenwasserstoffgemisch also direkt in den Schritt a) des erfindungsgemäßen Verfahrens oder aber erst nach einer Vorbehandlung durch eines oder mehrere der vorgenannten Verfahren eingesetzt werden.

Durch das erfindungsgemäße Verfahren können, je nach Zusammensetzung des Edukts I, als 1-Buten-haltige Fraktionen V und/oder VI Gemische, enthaltend über 98 Massen-% 1-Buten, vorzugsweise über 99,6 Massen-% und weniger als 2000 wppm Isobuten, vorzugsweise von 0 bis 1500 wppm, bevorzugt von 50 bis 500 wppm Isobuten hergestellt werden. Diese Gemische enthalten weniger als 0,05 Massen-% 2-Butene. Besonders bevorzugt enthalten die Gemische weniger als 5 Massen-ppm an organischen, sauerstoffhaltigen Verbindungen. Dies kann insbesondere dadurch erreicht werden, dass Etherverbindungen, wie Diethylether oder ETBE, in Verfahrensschritt b) abgetrennt werden und Ethanol durch entsprechende Extraktionen bzw. Wasserwäschen aus dem Gemisch entfernt werden. Ganz besonders bevorzugt enthalten die Gemische weniger als 50 Massen-ppm an Wasser. Dies kann insbesondere dadurch erreicht werden, dass das Wasser durch entsprechend häufige Trocknungen oder eine ausreichend dimensionierte Trocknung aus dem Gemisch entfernt wird.

Die Gemische, können z. B. als 1-Buten enthaltende Einsatzstoffe bei der Copolymerisation von Ethylen mit 1-Buten verwendet werden. Das Gemisch kann beispielsweise als Comonomer bei der Herstellung von Polyethylen (LLDPE oder HDPE) sowie von Ethylen-Propylen-Mischpolymeren eingesetzt werden. Es ist Ausgangsstoff für die Herstellung von Butan-2-ol, Butenoxid, Valeraldehyd. Eine weitere Verwendung des erfindungsgemäß hergestellten, nahezu Isobuten-freien, 1-Butens ist die Herstellung von n-ButenOligomeren, insbesondere nach dem Octol-Prozess. Optional können die erfindungsgemäß hergestellten Produkte, vorzugsweise nach Abtrennung vorhandener Sauerstoffverbindungen, für Alkylierungsreaktionen und zur Herstellung von Oligomeren verwendet werden.

### Beschreibung der Figuren

An Hand der Figuren Fig. 1 und 2 wird das erfindungsgemäße Verfahren nachfolgend näher erläutert, ohne dass das Verfahren auf die dort beispielhaft abgebildeten Ausführungsarten beschränkt sein soll. In den schematischen Darstellungen sind nur die wesentlichen Stufen dargestellt. Auf die Darstellung von verfahrenstechnisch üblichen Strömen, wie z. B. Kühlwasserströmen, Kreislaufströmen, Katalysatorrückführungen oder Rückspeisungen, und/oder üblichen Apparaturen, wie z. B. Wärmetauschern oder Abscheidern wurde teilweise zu Gunsten einer besseren Übersicht verzichtet.

Die Bezeichnungen in den Figuren Fig. 1 bis Fig. 6 haben folgende Bedeutungen:
I technische Mischung von C₄-Kohlenwasserstoffen enthaltend 1-Buten, 2000 wppm bis 3 Massen-% Isobuten und gegebenenfalls n-Butan, Isobutan und/oder 2-Butene.
II Reaktoraustrag aus dem letzten Veretherungsreaktor
III ETBE aufweisende Fraktion
IV 1-Buten und Ethanol sowie gegebenenfalls n-Butan, Isobutan und/oder 2- Butene aufweisende Fraktion
V An Ethanol verarmter Strom
VI 1-Buten Fraktion
VII n-Butan, Isobutan und/oder 2-Butene aufweisende Fraktion
   Et Ethanol

   D-d1 Kopfprodukt von K-d1
   D-d2 Kopfprodukt von K-d2 (Isobutanfraktion)
   E-c 1 Zulauf Extraktionsmittel
   E-c2 Ablauf Extraktionsmittel
   K-b1 Destillationskolonne
   K-c1 Extraktionskolonne
   K-d1 Destillationskolonne
   K-d2 Destillationskolonne

   R-a1 Reaktor
   R-a2 Reaktor

   W-b 1 Sumpfverdampfer
   W-b2 Kondensator
   W-d1 Sumpfverdampfer
   W-d2 Kondensator
   W-d3 Sumpfverdampfer
   W-d4 Kondensator

### Fig. 1a

Bei der in Fig. 1a schematisch dargestellten Variante des erfindungsgemäßen Verfahrens wird das technische Gemisch I zusammen mit Ethanol (Et) zunächst in einen ersten Veretherungsreaktor R-al, der bevorzugt als Schlaufenreaktor ausgeführt ist, gefahren. Das Produkt aus dem ersten Reaktor wird in einen zweiten Veretherungsreaktor R-a2 gefahren (Fahrweise mit gleicher oder unterschiedlicher Temperatur etc. möglich). Der Austrag II aus dem zweiten Veretherungsreaktor wird in eine Destillationskolonne K-b1 überführt, die mit einem Kondensator W-b2 für das Kopfprodukt und einem Sumpfverdampfer W-b1 ausgestattet ist. Ein Teil des Kopfproduktes wird als Rücklauf in die Kolonne zurückgefahren. Als Kopfprodukt wird der Strom IV abgenommen, der im Wesentlichen 1-Buten, n-Butan und Ethanol aufweist, und als Sumpfprodukt wird ein ETBE aufweisender Strom III erhalten. Das Kopfprodukt IV wird unten in eine Extraktionskolonne K-c1 gefahren, in die im Gegenstrom ein Extraktionsmittel, wie z. B. Wasser, über den am Kopf befindlichen Zulauf E-c1 eingespeist wird, welches über den Ablauf E-c2 am Sumpf der Kolonne entnommen wird. Am Kopf der Kolonne wird als Produkt der Extraktion ein Strom V erhalten, der an Ethanol abgereichert ist. Dieser Strom V wird seitlich in eine Destillationskolonne K-d1, die mit einem Sumpfverdampfer W-d1 und am Kopf mit einem Kondensator W-d2 und gegebenenfalls einem Dekanter ausgerüstet ist, eingespeist und in eine 2-Butene und n-Butan aufweisende Fraktion VII, die am Sumpf der Kolonne abgenommen wird, und eine nahezu Isobuten-freie 1-Buten aufweisende Fraktion VI, die gegebenenfalls in einem Dekanter von einer wässrigen Phase getrennt wird, aufgetrennt. Der Kopf der Kolonne K-d1 ist so ausgerüstet, dass ein Teil als Rücklauf in die Kolonne zurückgefahren werden kann.

### Fig. 1b

Die in Figur 1b dargestellte Anlage zur Durchführung des erfindungsgemäßen Verfahrens entspricht der Anlage gemäß Figur 1a. Der Unterschied besteht im Wesentlichem darin, dass in Kolonne K-d1 weder 2-Butene noch gegebenenfalls n-Butan vom 1-Buten, sondern Isobutan (Kopfprodukt) vom 1-Buten (Sumpfprodukt) abgetrennt werden/wird. Diese Fahrweise kann nur dann gewählt werden, wenn Strom V praktisch kein n-Butan oder keine 2-Butene aufweist, d. h., diese Stoffe dürfen weder im Edukt I enthalten sein noch dürfen 2-Butene durch Isomerisierung von 1-Buten entstehen.

### Fig. 2

In dieser Figur ist eine Variante der in Fig. 1a dargestellten Anlage dargestellt. Bei dieser Variante werden die Schritte a) bis c) in einer wie in Fig. 1a dargestellten Verschaltung durchgeführt. Der Schritt d) wird im Unterschied zu der Variante gemäß Fig. 1a in zwei Destillationskolonnen durchgeführt. Der aus der Extraktionskolonne K-c1 erhaltene, an Ethanol abgereicherte Strom V wird in die Destillationskolonne K-d1 überführt, in welcher eine nahezu Isobuten-freie 1-Buten-haltige Fraktion D-d1 über Kopf abgetrennt wird. Als Sumpfprodukt wird eine 2-Butene und n-Butan aufweisende Fraktion VII erhalten. Das Destillat D-d1 der Kolonne K-d1 wird direkt in eine weitere Kolonne K-d2 geführt, die mit einem Sumpfverdampfer W-d3 und am Kopf mit einem Kondensator W-d4 ausgerüstet ist, in welcher es in ein 1-Buten enthaltendes Sumpfprodukt VI und ein Isobutan und/oder Leichtsieder aufweisendes Kopfprodukt D-d2 getrennt wird.

Die folgenden Beispiele sollen die Erfindung erläutern, ohne den Schutzbereich der sich aus den Patentansprüchen und der Beschreibung ergibt, einzuschränken.

### Beispiele

Die nachfolgenden Beispielrechnungen wurden mit dem Simulationsprogramm ASPEN Plus durchgeführt. Um transparente, reproduzierbare Daten zu erzeugen, wurden nur allgemein zugängliche Stoffdaten eingesetzt. Außerdem wurde bei allen Varianten auf den Einsatz einer Reaktivdestillation verzichtet. Durch diese Vereinfachungen ist es dem Fachmann leicht möglich, die Berechnungen nachzuvollziehen. Die eingesetzten Methoden besitzen zwar keine ausreichende Genauigkeit für die Auslegung technischer Anlagen, die qualitativen Unterschiede der Schaltungen werden aber korrekt erfasst. In allen gezeigten Varianten konnte der Isobuten-Umsatz durch Einsatz einer oder mehrerer Reaktivdestillation(en) erhöht werden.

In den Beispielen wurde die Property-Methode "UNIFAC-DMD" (s. J. Gmehling, J. Li, and M. Schiller, Ind. Eng. Chem. Res. 32, (1993), pp. 178-193) benutzt. Folgende Annahmen wurden getroffen:
- Für den Reaktor R-a1 wurde jeweils ein Katalysatorvolumen von 10 1 angenommen, für den Reaktor R-a2 wurde jeweils ein Reaktorvolumen von 7,5 1 angenommen. Beide Reaktoren waren mit dem Ionentauscher Amberlyst^{®} 15 befüllt. Für die Reaktormodellierung wurde in den Rechnungen ein kinetisches Reaktormodell verwendet, das auf umfangreichen experimentellen Messdaten beruht. In den Beispielen werden daher jeweils auch die Reaktionstemperaturen genannt, die bei der Reaktormodellierung angenommen wurden. Da auch jeweils die Zusammensetzung der ein- und ausgehenden Ströme der Reaktionsstufe genannt werden, ist es dem Fachmann durch Nachstellung der Reaktoren mit fest vorgegebenen Umsätzen möglich, das Beispiel nachzurechnen, ohne die genauen Gleichungen für die Kinetik zu kennen. Der Reaktordruck betrug in allen Beispielen 13 bar(abs).
- In den ETBE-Kolonnen wurde über Kopf ein C₄-/Ethanol-Azeotrop abgetrennt. Das EtOH wurde in Extraktoren mit Wasser ausgewaschen, die als einfache Komponenten-Splitter modelliert wurden.
- Das aus den Extraktoren erhaltene EtOH-Wasser-Gemisch wurde in einer weiteren Kolonne K-EtOH destillativ aufgearbeitet, die in den Schaltbildern nicht dargestellt wurde. Beide Produkte der K-EtOH können - gegebenenfalls nach einer geeigneten Trocknung des Ethanols - in den Prozess rezirkuliert werden.

In allen Beispielen sollte das im C4-Rohstoffstrom (I) zum Reaktor R-a1 (s. Fig. 1a, 1b bzw. 2) enthaltene Isobuten durch ETBE-Synthese chemisch abgetrennt und 1-Buten mit einer Reinheit größer 99,6 Massen-% hergestellt werden. Im 1-Buten-Produkt sollten maximal 2000 ppm Isobuten, 500 ppm 2-Butene und 2000 ppm Butane vorliegen (s. Tabelle 1).

**Tabelle 1: Geforderte 1-Buten-Spezifikation (in Massen-%).**

| | 1-Buten-Spezifikation |
|---|---|
| Komponenten [%] | |
| Isobutan + n-Butan | < 0,2000 |
| Isobuten | < 0,2000 |
| 1-Buten | > 99,6000 |
| trans-2-Buten + cis-2-Buten | < 0,0500 |

### Beispiel 1a:

Das Beispiel 1a entspricht der in Fig. 1a dargestellten Variante. Als Zulauf zu dem Reaktor R-a1 wurde gemäß Fig. 1a ein Rohstoffstrom (I) von 10 kg/h mit 8 Massen-% n-Butan, 2 Massen-% Isobuten und 90 Massen-% 1-Buten und ein Ethanolstrom von 3 kg/h angenommen (s. Tabelle 2)

**Tabelle 2: Zusammensetzung des C₄-Eintrittstroms (I) sowie des Ethanol-Eintrittstroms (Et) für Beispiel 1a (in Massen-%).**

| | | C₄-Feed (I) | Ethanol (Et) |
|---|---|---|---|
| Massenstrom [kg/h] | | 10,000 | 3,000 |
| Komponenten [%] | | | |
| | Isobuten | 2,0000 | |
| | 1-Buten | 90,000 | |
| | n-Butan | 8,0000 | |
| | trans-2-Buten | | |
| | cis-2-Buten | | |
| | ETBE | | |
| | Ethanol | | 100,0000 |

Mit einer Reaktionstemperatur von 46 °C für R-a1 und 40°C für R-a2 ergab sich für Strom II die in Tabelle 3 aufgeführte Zusammensetzung. In der Destillationsstufe K-b1 wurde das ETBE als Sumpfprodukt (III) abgetrennt. Die Kolonne hatte 50 theoretische Stufen und wurde bei einem Rücklaufverhältnis von 0,9 und bei einem Druck von 6 bar(abs) betrieben. Der Zulauf erfolgte oberhalb Stufe 30. Das Destillat dieser Kolonne (IV) war ein C₄/EtOH-Azeotrop, aus dem das Ethanol mit Wasser in der Extraktionskolonne K-f1 ausgewaschen wurde.

**Tabelle 3: Zusammensetzung der Ein- und Austrittströme der Kolonne K-b1 (II, III und IV) für Beispiel 1a (in Massen-%).**

| | | Zulauf K-b1 (II) | Sumpfprodukt K-b1 (III) | Destillat K-b 1 (IV) |
|---|---|---|---|---|
| Massenstrom [kg/h] | | 13,000 | 3,055 | 9,945 |
| Komponenten [%] | | | | |
| | Isobuten | 0,1364 | | 0,1783 |
| | 1-Buten | 69,1938 | | 90,4494 |
| | n-Butan | 6,1538 | | 8,0442 |
| | trans-2-Buten | 0,0185 | | 0,0242 |
| | cis-2-Buten | 0,0185 | | 0,0242 |
| | ETBE | 2,5533 | 10,8649 | |
| | Ethanol | 21,9257 | 89,1351 | 1,2797 |

Das ethanolfreie Raffinat (V) der Extraktionskolonne K-c1 wurde einer C₄-Kolonne K-d1 zugeführt, in der vornehmlich n-Butan und 2-Butene über Sumpf (VII) abgetrennt wurden. Die Kolonne hatte 150 theoretische Stufen und wurde bei einem Rücklaufverhältnis von 19 und bei einem Druck von 8 bar(abs) betrieben. Der Kolonnenzulauf erfolgte oberhalb Stufe 85. Als Kopfprodukt (VI) wurde eine Fraktion erhalten, die über 99,6 Massen-% 1-Buten enthielt und der in Tabelle 1 geforderten Spezifikation für das 1-Buten-Produkt genügt (s. Tabelle 4).

**Tabelle 4: Zusammensetzung der Ein- und Austrittströme der Kolonne K-d1 (V, VI und VII) für Beispiel 1a (in Massen-%).**

| | | Zulauf K-d1 (V) | Destillat K-d1 (VI) | Sumpfprodukt K-d1 (VII) |
|---|---|---|---|---|
| Massenstrom [kg/h] | | 9,818 | 8,998 | 0,820 |
| Komponenten [%] | | | | |
| | Isobuten | 0,1806 | 0,1968 | 0,0029 |
| | 1-Buten | 91,6217 | 99,7000 | 3,0002 |
| | n-Butan | 8,1485 | 0,1032 | 96,4089 |
| | trans-2-Buten | 0,0246 | | 0,2939 |
| | cis-2-Buten | 0,0246 | | 0,2939 |
| | ETBE | | | 0,0002 |
| | Ethanol | | | |

### Beispiel 1b:

Die nachfolgende Beispielrechnung entspricht der in Fig. 1b dargestellten Verfahrensvariante. Als Zulauf zu dem Reaktor R-a1 gemäß Fig. 1b wurde ein Rohstoffstrom (I) von 10 kg/h mit 4 Massen-% Isobutan, 1 Massen-% Isobuten und 95 Massen-% 1-Buten und ein Ethanolstrom von 1,5 kg/h angenommen (s. Tabelle 5)

**Tabelle 5: Zusammensetzung des C₄-Eintrittstroms (I) sowie des Ethanol-Eintrittstroms (Et) für Beispiel 1b (in Massen-%).**

| | | C₄-Feed (I) | Ethanol (Et) |
|---|---|---|---|
| Massenstrom [kg/h] | | 10,000 | 1,500 |
| Komponenten [%] | | | |
| | Isobutan | 4,0000 | |
| | Isobuten | 1,0000 | |
| | 1-Buten | 95,0000 | |
| | Trans-2-Buten | | |
| | cis-2-Buten | | |
| | ETBE | | |
| | Ethanol | | 100,0000 |

Mit einer Reaktionstemperatur von 41,5 °C für R-a1 und 35 °C für R-a2 ergab sich für Strom II die in Tabelle 6 aufgeführte Zusammensetzung. In der Destillationsstufe K-b1 wurde das ETBE als Sumpfprodukt (III) abgetrennt. Die Stufenzahl, Druck und Rücklaufverhältnis waren gegenüber Beispiel 1a unverändert. Das Destillat dieser Kolonne (IV) war wieder ein C₄/EtOH-Azeotrop, aus dem das Ethanol mit Wasser in der Extraktionskolonne K-f1 ausgewaschen wurde.

**Tabelle 6: Zusammensetzung der Ein- und Austrittströme der Kolonne K-b1 (II, III und IV) für Beispiel 1b (in Massen-%).**

| | | Zulauf K-b1 (II) | Sumpfprodukt K-b1 (III) | Destillat K-b1 (IV) |
|---|---|---|---|---|
| Massenstrom [kg/h] | | 11,500 | 1,465 | 10,035 |
| Komponenten [%] | | | | |
| | Isobutan | 3,4783 | | 3,9862 |
| | Isobuten | 0,1612 | | 0,1847 |
| | 1-Buten | 82,5744 | | 94,6337 |
| | trans-2-Buten | 0,0171 | | 0,0195 |
| | cis-2-Buten | 0,0171 | | 0,0195 |
| | ETBE | 1,2900 | 10,1233 | |
| | Ethanol | 12,4619 | 89,8767 | 1,1564 |

Das ethanolfreie Raffinat (V) der Extraktionskolonne K-c1 wurde einer C₄-Kolonne K-d1 zugeführt, in der vornehmlich Isobutan über Kopf (VII) abgetrennt wurde. Stufenzahl und Betriebsdruck der Kolonne entsprachen Beispiel 1a, das Rücklaufverhältnis war auf 184 eingestellt. Als Sumpfprodukt (VI) wurde eine Fraktion erhalten, die über 99,6 Massen-% 1-Buten enthält und der in Tabelle 1 geforderten Spezifikation für das 1-Buten-Produkt genügt (s. Tabelle 7).

**Tabelle 7: Zusammensetzung der Ein- und Austrittströme der Kolonne K-d1 (V, VI und VII) für Beispiel 1b (in Massen-%).**

| | | Zulauf K-d1 (V) | Destillat K-d1 (VII) | Sumpfprodukt K-d1 (VI) |
|---|---|---|---|---|
| Massenstrom [kg/h] | | 9,919 | 0,415 | 9,504 |
| Komponenten [%] | | | | |
| | Isobutan | 4,0329 | 94,9842 | 0,0644 |
| | Isobuten | 0,1869 | 0,0158 | 0,1944 |
| | 1-Buten | 95,7408 | 5,0000 | 99,7000 |
| | trans-2-Buten | 0,0198 | | 0,0206 |
| | cis-2-Buten | 0,0198 | | 0,0206 |
| | ETBE | | | |
| | Ethanol | | | |

### Beispiel 2:

Die nachfolgende Beispielrechnung entspricht der in Fig. 2 dargestellten Verfahrensvariante. Für Beispiel 2 wurde als Zulauf zu dem Reaktor R-a1 gemäß Fig. 2 ein Rohstoffstrom (I) von 10 kg/h mit 5 Massen-% Isobutan, 2 Massen-% Isobuten, 61 Massen-% 1-Buten, 10 Massen-% n-Butan, 8 Massen-% trans-2-Buten und 14 Massen-% cis-2-Buten und ein Ethanolstrom von 1,5 kg/h angenommen (s. Tabelle 8).

**Tabelle 8: Zusammensetzung des C₄-Eintrittstroms (I) sowie des Ethanol-Eintrittstroms (Et) für Beispiel 2 (in Massen-%).**

| | | C₄-Feed (I) | Ethanol (Et) |
|---|---|---|---|
| Massenstrom [kg/h] | | 10,000 | 1,500 |
| Komponenten [%] | | | |
| | Isobutan | 5,0000 | |
| | Isobuten | 2,0000 | |
| | 1-Buten | 61,0000 | |
| | n-Butan | 10,0000 | |
| | trans-2-Buten | 8,0000 | |
| | cis-2-Buten | 14,0000 | |
| | ETBE | | |
| | Ethanol | | 100,0000 |

Mit einer Reaktionstemperatur von 50 °C für R-a1 und 42 °C für R-a2 ergab sich für Strom II die in Tabelle 9 aufgeführte Zusammensetzung. In der Destillationsstufe K-b1 wurde das ETBE als Sumpfprodukt (III) abgetrennt. Die Stufenzahl, Druck und Rücklaufverhältnis waren gegenüber Beispiel 1a unverändert. Das Destillat dieser Kolonne (IV) war wieder ein C₄/EtOH-Azeotrop, aus dem das Ethanol mit Wasser in der Extraktionskolonne K-f1 ausgewaschen wurde

**Tabelle 9: Zusammensetzung der Ein- und Austrittströme der Kolonne K-b1 (II, III und IV) für Beispiel 2 (in Massen-%).**

| | | Zulauf K-b1 (II) | Sumpfprodukt K-b1 (III) | Destillat K-b1 (IV) |
|---|---|---|---|---|
| Massenstrom [kg/h] | | 11,500 | 1,534 | 9,966 |
| Komponenten [%] | | | | |
| | Isobutan | 4,3478 | | 5,0170 |
| | Isobuten | 0,0457 | | 0,0528 |
| | 1-Buten | 52,9649 | | 61,1167 |
| | n-Butan | 8,6957 | | 10,0340 |
| | trans-2-Buten | 6,9958 | | 8,0726 |
| | cis-2-Buten | 12,2132 | | 14,0930 |
| | ETBE | 3,0838 | 23,1198 | |
| | Ethanol | 11,6531 | 76,8802 | 1,6137 |

Das ethanolfreie Raffinat (V) der Extraktionskolonne K-c1 wurde einer C₄-Kolonne K-d1 zugeführt, in der n-Butan, 2-Buten und ca. 25 % des 1-Butens als Sumpfprodukt (VII) abgetrennt wurden (Zusammensetzung s. Tabelle 10). Die Kolonne hatte 160 theoretische Stufen und wurde bei einem Rücklaufverhältnis von 18 und bei einem Druck von 8 bar(abs) betrieben. Die Feedzugabe erfolgte oberhalb Stufe 80. Das vornehmlich aus 1-Buten und Isobutan bestehende Destillat (D-d1) wurde zur Aufreinigung des 1-Buten einer weiteren Destillationskolonne K-d2 zugeführt, wobei das Isobutan über Kopf (D-d2) abgetrennt wurde. Die Destillationskolonne K-d2 hat 150 theoretische Stufen und wurde bei einem Rücklaufverhältnis von 85 und bei einem Druck von 8 bar(abs) betrieben. Die Feedzugabe erfolgte oberhalb Stufe 75. Als Sumpfprodukt (VI) wurde eine Fraktion erhalten, die über 99,6 Massen-% 1-Buten enthält und der in Tabelle 2 geforderten Spezifikation für das 1-Buten-Produkt genügt (s. Tabelle 10).

**Tabelle 10: Zusammensetzung der Ein- und Austrittströme der Kolonnen K-d1 (V, K-d1 und VII) und Kolonne K-d2 (D-d2 und VI) für Beispiel 2 (in Massen-%).**

| | | Zulauf K-d 1 (V) | Sumpfprodukt K-d1 (VII) | Destillat K-d1 (D-d1) | Destillat K-d2 (D-d2) | Sumpfprodukt K-d2 (VI) |
|---|---|---|---|---|---|---|
| Massenstrom [kg/h] | | 9,805 | 4,725 | 5,080 | 0,533 | 4,547 |
| Komponenten [%] | | | - | | | |
| | Isobutan | 5,0993 | 0,0006 | 9,8417 | 93,5384 | 0,0323 |
| | Isobuten | 0,0536 | 0,0139 | 0,0905 | 0,0100 | 0,1000 |
| | 1-Buten | 62,1193 | 32,2323 | 89,9177 | 6,4516 | 99,7000 |
| | n-Butan | 10,1986 | 21,0022 | 0,1500 | | 0,1676 |
| | trans-2-Buten | 8,2050 | 17,0264 | 0,0001 | | 0,0001 |
| | cis-2-Buten | 14,3242 | 29,7246 | | | |
| | ETBE | | - | | | |
| | Ethanol | | - | | | |

## Patentansprüche

1. Verfahren zur Herstellung einer 1-Buten-haltigen Fraktion, umfassend:
- über 99,6 Massen-% 1-Buten
- weniger als 0,2 Massen-% Isobuten,
- weniger als 0,05 Massen-% 2-Butene,
aus technischen Mischungen von C₄-Kohlenwasserstoffen I, die mindestens 1-Buten und von 2000 wppm bis 8 Massen-% Isobuten bezogen auf den Gehalt an 1-Buten enthalten, **gekennzeichnet durch** die Verfahrensschritte
a) Umsetzung zumindest eines Teils des in der technischen Mischung I enthaltenen Isobutens mit Ethanol in einer Reaktionszone oder einer Serie von zumindest zwei hintereinandergeschalteten Reaktionszonen in Gegenwart eines sauren Katalysators zu Ethyl-tert.-butylether,
b) Überführung des Reaktoraustrags II des letzten Reaktors der Serie in eine thermische Abtrennung, in der eine Ethyl-tert.-butylether aufweisende Fraktion III und eine Fraktion IV erhalten wird, die 1-Buten und Ethanol aufweist und
c) Abtrennung des Ethanols aus der Fraktion IV unter Erhalt eines 1-Buten-haltigen Fraktion V, und optional
d) Abtrennung von gegebenenfalls in der Fraktion IV enthaltenen C₄-Kohlenwasserstoffen, die nicht 1-Buten oder Isobuten sind, in zumindest einem weiteren Trennschritt unter Erhalt einer 1-Buten-haltigen Fraktion VI
wobei die Umsetzung in der ersten Reaktionszone in Schritt a) mit einem zumindest dreifachen molaren Überschuss an Ethanol in Bezug auf das in Mischung I enthaltene Isobuten durchgeführt wird.

2. Verfahren nach Anspruch 1
**dadurch gekennzeichnet,**
**dass** eine technische Mischung von C₄-Kohlenwasserstoffen I eingesetzt wird, die einen Anteil an 1-Buten von größer 50 Massen-% aufweist.

3. Verfahren nach zumindest einem der Ansprüche 1 bis 2,
**dadurch gekennzeichnet,**
**dass** die sauer katalysierte Veretherung in Stufe a) so durchgeführt wird, dass zumindest eine Reaktionszone als Reaktivdestillation ausgeführt wird.

4. Verfahren nach zumindest einem der Ansprüche 1 bis 3,
**dadurch gekennzeichnet,**
**dass** die sauer katalysierte Veretherung in Stufe a) so durchgeführt wird, dass die letzte Reaktionszone als Reaktivdestillation ausgeführt ist, in der auch der Schritt b) durchgeführt wird.

5. Verfahren nach zumindest einem der Ansprüche 1 bis 2,
**dadurch gekennzeichnet,**
**dass** die sauer katalysierte Veretherung in Stufe a) so durchgeführt wird, dass keine Reaktionszone als Reaktivdestillation ausgeführt wird.

6. Verfahren nach zumindest einem der Ansprüche 1 bis 5,
**dadurch gekennzeichnet,**
**dass** in Stufe c) eine Extraktion durchgeführt wird und als Extraktionsmittel Wasser oder eine wässrige Lösung eingesetzt wird.

7. Verfahren nach zumindest einem der Ansprüche 1 bis 6,
**dadurch gekennzeichnet,**
**dass** in den Fraktionen V oder VI vorhandene Restmengen an Ethanol in einem weiteren Extraktionsschritt mit Wasser ausgewaschen werden.

8. Verfahren nach zumindest einem der Ansprüche 1 bis 7,
**dadurch gekennzeichnet,**
**dass** eine technische Mischung I eingesetzt wird, die einen Anteil an mehrfach ungesättigten Verbindungen von kleiner-gleich 20 Massen-ppm aufweist.

9. Verfahren nach zumindest einem der Ansprüche 1 bis 8,
**dadurch gekennzeichnet,**
**dass** in den C₄-Kohlenwasserstoffströmen enthaltene mehrfach ungesättigten Kohlenwasserstoffe in einer zusätzlichen Reinigungsstufe, die einer oder mehreren der Verfahrensschritte a), b), c) oder d) vorgeschaltet wird, katalytisch hydriert werden.

10. Verfahren nach Anspruch 9,
**dadurch gekennzeichnet,**
**dass** die Hydrierung der mehrfach ungesättigten Verbindungen in mindestens zwei Reaktionsstufen erfolgt, wobei mindestens die letzte Reaktionsstufe in Anwesenheit von 0,05 bis 100 wppm CO durchgeführt wird.

11. Verfahren nach zumindest einem der Ansprüche 1 bis 10,
**dadurch gekennzeichnet,**
**dass** als saurer Katalysator ein Ionentauscherharz eingesetzt wird.

12. Verfahren nach zumindest einem der Ansprüche 1 bis 11,
**dadurch gekennzeichnet,**
**dass** die Umsetzung in Schritt a) bei einer Temperatur von 30 bis 70 °C durchgeführt wird.

13. Verfahren nach zumindest einem der Ansprüche 1 bis 12,
**dadurch gekennzeichnet,**
**dass** die Umsetzung in Schritt a) bei einer Temperatur in zumindest einer Reaktionszone von 30 bis 50 °C durchgeführt wird und ein Katalysator eingesetzt wird, der eine Säurekapazität von größer 3 bis 5,5 mol/kg aufweist oder dass die Umsetzung in Schritt a) bei einer Temperatur von 51 bis 70 °C durchgeführt wird und ein Katalysator eingesetzt wird, der eine Säurekapazität von 0,5 bis 3 mol/kg aufweist.

14. Verfahren nach zumindest einem der Ansprüche 1 bis 13,
**dadurch gekennzeichnet,**
**dass** die Umsetzung im Verfahrensschritt a) vor dem Destillationsschritt b) mit einer LHSV von 0,3 bis 2,5 m³/(m³_{KAT}h) durchgeführt wird.

15. Verfahren nach zumindest einem der Ansprüche 1 bis 14,
**dadurch gekennzeichnet,**
**dass** die Umsetzung in der ersten Reaktionszone in Schritt a) mit einem 10- bis 15-fachen Überschuss an Ethanol in Bezug auf das in Mischung I enthaltene Isobuten durchgeführt wird.

## Claims

1. A process for preparing a 1-butenic fraction comprising:
- more than 99.6% by mass of 1-butene,
- less than 0.2% by mass of isobutene,
- less than 0.05% by mass of 2-butenes,
from technical mixtures of C₄ hydrocarbons I which contain at least 1-butene and from 2000 ppmw to 8% by mass of isobutene based on the content of 1-butene, **characterized by** the process steps of
a) reacting at least a portion of the isobutene present in the technical mixture I with ethanol in a reaction zone or a series of at least two reaction zones connected in series in the presence of an acidic catalyst to give ethyl tert-butyl ether,
b)transferring the reactor effluent II of the last reactor of the series into a thermal removal in which a fraction III comprising ethyl tert-butyl ether and a fraction IV which comprises 1-butene and ethanol are obtained and
c) removing the ethanol from fraction IV to obtain a 1-butenic fraction V, and optionally
d)removing any C₄ hydrocarbons other than 1-butene or isobutene which are present in the fraction IV in at least one further separating step to obtain a 1-butenic fraction VI,
the reaction in the first reaction zone in step a) being carried out with an at least threefold molar excess of ethanol based on the isobutene present in mixture I.

2. A process according to claim 1,
**characterized in that**
a technical mixture of C₄ hydrocarbons I which has a content of 1-butene of greater than 50% by mass is used.

3. A process according to at least one of claims 1 and 2,
**characterized in that**
the acid-catalyzed etherification in stage a) is carried out in such a way that at least one reaction zone is designed as a reactive distillation.

4. A process according to at least one of claims 1 to 3,
**characterized in that**
the acid-catalyzed etherification in stage a) is carried out in such a way that the last reaction zone is designed as a reactive distillation, in which step b) is also carried out.

5. A process according to at least one of claims 1 and 2,
**characterized in that**
the acid-catalyzed etherification in stage a) is carried out in such a way that no reaction zone is designed as a reactive distillation.

6. A process according to at least one of claims 1 to 5,
**characterized in that**
an extraction is carried out in stage c) and the extractant used is water or an aqueous solution.

7. A process according to at least one of claims 1 to 6,
**characterized in that**
residual amounts of ethanol present in fractions V or VI are scrubbed out with water in a further extraction step.

8. A process according to at least one of claims 1 to 7,
**characterized in that**
a technical mixture I which has a content of polyunsaturated compounds of less than or equal to 20 ppm by mass is used.

9. A process according to at least one of claims 1 to 8,
**characterized in that**
polyunsaturated hydrocarbons present in the C₄ hydrocarbon streams are catalytically hydrogenated in an additional purification stage which is inserted upstream of one or more of process steps a), b), c) or d).

10. A process according to claim 9,
**characterized in that**
the polyunsaturated compounds are hydrogenated in at least two reaction stages, at least the last reaction stage being carried out in the presence of 0.05 to 100 ppmw of CO.

11. A process according to at least one of claims 1 to 10,
**characterized in that**
the acidic catalyst used is an ion exchange resin.

12. A process according to at least one of claims 1 to 11,
**characterized in that**
the reaction in step a) is carried out at a temperature of 30 to 70 °C.

13. A process according to at least one of claims 1 to 12,
**characterized in that**
the reaction in step a) is carried out at a temperature in at least one reaction zone of 30 to 50°C and a catalyst which has an acid capacity of greater than 3 to 5.5 mol/kg is used, or **in that** the reaction in step a) is carried out at a temperature of 51 to 70°C and a catalyst which has an acid capacity of 0.5 to 3 mol/kg is used.

14. A process according to at least one of claims 1 to 13,
**characterized in that**
the reaction in process step a) is carried out before the distillation step b) with an LHSV of 0.3 to 2.5 m³/ (m³_{CAT}h) .

15. A process according to at least one of claims 1 to 14,
**characterized in that**
the reaction in the first reaction zone in step a) is carried out with a 10- to 15-fold excess of ethanol in relation to the isobutene present in the mixture I.

## Revendications

1. Procédé pour la préparation d'une fraction contenant du 1-butène, comprenant
- plus de 99,6% en masse de 1-butène,
- moins de 0,2% en masse d'isobutène,
- moins de 0,05% en masse de 2-butène,
à partir de mélanges techniques I d'hydrocarbures en C₄, qui contiennent au moins du 1-butène et de 2000 wppm (ppm en masse) à 8% en masse d'isobutène par rapport à la teneur en 1-butène, **caractérisé par** les étapes de procédé
a) transformation d'au moins une partie de l'isobutène contenu dans le mélange technique I avec de l'éthanol dans une zone de réaction ou une série d'au moins deux zones de réaction disposées l'une derrière l'autre en présence d'un catalyseur acide en éthyl-tert-butyléther,
b) transfert du produit II sortant du dernier réacteur de la série dans une séparation thermique, dans laquelle une fraction III présentant de l'éthyl-tert-butyléther et une fraction IV, qui présente du 1-butène et de l'éthanol sont obtenues et
c) séparation de l'éthanol de la fraction IV avec obtention d'une fraction V contenant du 1-butène, et éventuellement
d) séparation d'hydrocarbures en C₄ le cas échéant contenus dans la fraction IV, qui ne sont pas du 1-butène ou de l'isobutène, dans au moins une autre étape de séparation, avec obtention une fraction VI contenant du 1-butène
la transformation dans la première zone de réaction dans l'étape a) étant réalisée avec au moins un triple excès molaire d'éthanol par rapport à l'isobutène contenu dans le mélange I.

2. Procédé selon la revendication 1, **caractérisé en ce qu'**on utilise un mélange technique I d'hydrocarbures en C₄, qui présente une proportion de 1-butène supérieure à 50% en masse.

3. Procédé selon au moins l'une quelconque des revendications 1 à 2, **caractérisé en ce que** l'éthérification catalysée par voie acide dans l'étape a) est réalisée de manière telle qu'au moins une zone de réaction est réalisée sous forme de distillation réactive.

4. Procédé selon au moins l'une quelconque des revendications 1 à 3, **caractérisé en ce que** l'éthérification catalysée par voie acide dans l'étape a) est réalisée de manière telle que la dernière zone de réaction est réalisée sous forme de distillation réactive, dans laquelle l'étape b) est également réalisée.

5. Procédé selon au moins l'une quelconque des revendications 1 à 2, **caractérisé en ce que** l'éthérification catalysée par voie acide dans l'étape a) est réalisée de manière telle qu'aucune zone de réaction n'est réalisée sous forme de distillation réactive.

6. Procédé selon au moins l'une quelconque des revendications 1 à 5, **caractérisé en ce qu'**une extraction est réalisée dans l'étape c) et de l'eau ou une solution aqueuse est utilisée comme agent d'extraction.

7. Procédé selon au moins l'une quelconque des revendications 1 à 6, **caractérisé en ce que** des quantités résiduelles d'éthanol présentes dans les fractions V ou VI sont éliminées par lavage dans une autre étape d'extraction avec de l'eau.

8. Procédé selon au moins l'une quelconque des revendications 1 à 7, **caractérisé en ce qu'**on utilise un mélange technique I, qui présente une proportion de composés polyinsaturés inférieure ou égale à 20 ppm en masse.

9. Procédé selon au moins l'une quelconque des revendications 1 à 8, **caractérisé en ce que** les hydrocarbures polyinsaturés contenus dans les flux d'hydrocarbures en C₄ sont hydrogénés catalytiquement dans une étape de purification supplémentaire, qui est précédée d'une ou de plusieurs étapes de procédé a), b), c) ou d).

10. Procédé selon la revendication 9, **caractérisé en ce que** l'hydrogénation des composés polyinsaturés a lieu dans au moins deux étapes de réaction, au moins la dernière étape de réaction étant réalisée en présence de 0,05 à 100 wppm de CO.

11. Procédé selon au moins l'une quelconque des revendications 1 à 10, **caractérisé en ce qu'**on utilise une résine échangeuse d'ions comme catalyseur acide.

12. Procédé selon au moins l'une quelconque des revendications 1 à 11, **caractérisé en ce que** la transformation dans l'étape a) est réalisée à une température de 30 à 70°C.

13. Procédé selon au moins l'une quelconque des revendications 1 à 12, **caractérisé en ce que** la transformation dans l'étape a) est réalisée à une température, dans au moins une zone de réaction, de 30 à 50°C et un catalyseur est utilisé, qui présente une capacité d'acide supérieure à 3 à 5,5 moles/kg ou **en ce que** la transformation dans l'étape a) est réalisée à une température de 51 à 70°C et un catalyseur est utilisé, qui présente une capacité d'acide de 0,5 à 3 moles/kg.

14. Procédé selon au moins l'une quelconque des revendications 1 à 13, **caractérisé en ce que** la transformation dans l'étape de procédé a) est réalisée avant l'étape de distillation b) à une LHSV (liquid hourly space velocity - vitesse spatiale horaire de la phase liquide) de 0,3 à 2,5 m³ / (m³_{CAT}h) .

15. Procédé selon au moins l'une quelconque des revendications 1 à 14, **caractérisé en ce que** la transformation dans la première zone de réaction dans l'étape a) est réalisée avec un excès d'un facteur 10 à 15 d'éthanol par rapport à l'isobutène contenu dans le mélange I.
